(19) Europäisches Patentamt
European Patent Office
Office européen des brevets

(11) EP 1 828 172 B1

(12) **EUROPEAN PATENT SPECIFICATION**

(45) Date of publication and mention of the grant of the patent:
**25.11.2009 Bulletin 2009/48**

(21) Application number: **05815747.0**

(22) Date of filing: **12.12.2005**

(51) Int Cl.:
*C07D 407/04* (2006.01)
*C07D 401/04* (2006.01)
*C07D 207/34* (2006.01)
*A61K 31/40* (2006.01)
*A61K 31/4439* (2006.01)
*A61P 11/00* (2006.01)

(86) International application number:
**PCT/EP2005/013297**

(87) International publication number:
**WO 2006/063763 (22.06.2006 Gazette 2006/25)**

(54) **PYRROLE DERIVATIVES HAVING CRTH2 RECEPTOR ANTAGONIST ACTIVITY**

PYRROLDERIVATE MIT ANTAGONISTISCHER WIRKUNG AM CRTH2-REZEPTOR

DERIVES DE PYRROLE PRESENTANT UNE ACTIVITE ANTAGONISTE ENVERS LE RECEPTEUR CRTH2

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.12.2004 GB 0427381**

(43) Date of publication of application:
**05.09.2007 Bulletin 2007/36**

(73) Proprietors:
- **Novartis AG**
**4056 Basel (CH)**
Designated Contracting States:
**BE BG CH CY CZ DE DK EE ES FI FR GB GR HU IE IS IT LI LT LU LV MC NL PL PT RO SE SI SK TR**
- **Novartis Pharma GmbH**
**1230 Wien (AT)**
Designated Contracting States:
**AT**

(72) Inventors:
- **ADCOCK, Claire**
**Horsham,**
**West Sussex RH12 5AB (GB)**
- **LEBLANC, Catherine**
**Horsham,**
**West Sussex RH12 5AB (GB)**
- **SANDHAM, David Andrew**
**Horsham,**
**West Sussex RH12 5AB (GB)**
- **WATSON, Simon James**
**Horsham,**
**West Sussex RH12 5AB (GB)**
- **BALA, Kamlesh Jagdis**
**Horsham,**
**West Sussex RH12 5AB (GB)**

(74) Representative: **Bridle, Andrew Barry et al**
**Bridle Intellectual Property Ltd**
**6F Thomas Way**
**Lakeview International Business Park**
**Canterbury**
**Kent CT3 4JZ (GB)**

(56) References cited:
**EP-A- 0 182 737**
**EP-A- 1 466 902**
**WO-A-03/101981**
**WO-A-2004/089885**
**WO-A-2004/089913**
**WO-A-2005/116001**
**US-A- 5 037 847**

- **CHOLLET J-F ET AL: "Synthesis and phloem mobility of acidic derivatives of the fungicide fenpiclonil" PEST MANAGEMENT SCIENCE, vol. 60, September 2004 (2004-09), pages 1063-1072, XP002367075**
- **DATABASE CAPLUS CHEMICAL ABSTRACTS SERVICE, COLUMBUS, OHIO, US; XP002367152 retrieved from STN accession no. 2002:673868 Database accession no. 138:82915 & ZAHER N ET AL: JOURNAL OF ENZYME INHIBITION AND MEDICINAL CHEMISTRY, vol. 17, no. 2, 2002, pages 131-135,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 828 172 B1

- BOBERG F ET AL: "Heterocyclen aus alpha-Nitroolefin. X [1] Verseifung von 3-Pyrrolcarbonsäureestern mit Acetessigsäure- und Malonsäurederivaten als Stickstoffsubstituenten" J. HETEROCYCLIC CHEM., vol. 23, 1986, pages 753-757, XP002367076
- CHOLLET J-F: "Acidic derivatives o fthe fungicide fenpiclonil: effect of adding a methyl group to the N-substituted chain on systemicity and fungicidal activity" PEST MANAGEMENT SCIENCE, vol. 61, April 2005 (2005-04), pages 377-382, XP002367077

## Description

The present invention relates to organic compounds, their preparation and their use as pharmaceuticals.

In a first aspect, the present invention provides compounds of formula (I)

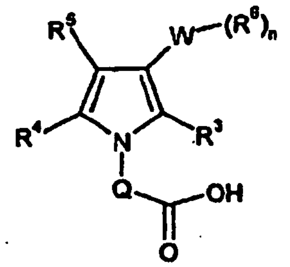

(I)

in free or pharmaceutically acceptable salt form, wherein
Q is

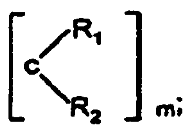

$R^1$ and $R^2$ are, independently, H, $C_1$-$C_6$-alkyl, or together with the carbon atom to which they are attached, form a divalent $C_3$-$C_8$-cycloaliphatic group;
$R^3$ and $R^4$ are independently selected from H and $C_1$-$C_8$-alkyl;
$R^5$ is cyano,
$R^6$ is selected from H, halogen, $C_1$-$C_8$-alkyl, $C_1$-$C_8$-haloalkyl, a $C_3$-$C_{15}$-carbocyclic group, nitro, cyano, $SO_2R^{6a}$, $C_1$-$C_8$alkylcarbonyl. $C_1$-$C_8$-alkoxycarbonyl, $C_1$-$C_8$-alkoxy, $C_1$-$C_8$-haloalkoxy, -$SR^{6b}$, carboxyl carboxy-$C_1$-$C_8$-alkyl, amino, amino($C_1$-$C_8$-alkyl), $C_1$-$C_8$-alkylamino($C_1$-$C_8$-alkyl), di($C_1$-$C_8$-alkyl)amino($C_1$-$C_8$-alkyl), $C_1$-$C_8$-alkylamino, di ($C_1$-$C_6$-alkyl)amino, $SO_2NR^{6c}R^{8d}$, -C(O)$NR^{6e}R^{6f}$, $C_1$-$C_8$-hydroxyalkyl, $NR^{6g}SO_2R^{6h}$, $NR^{6i}$(CO)$R^{6j}$, $SOR^{6k}$, a $C_6$-$C_{15}$-aromatic carbocyclic group, and a 4- to 10-membered heterocyclic group having one or more heteroatoms selected from the group consisting of oxygen, nitrogen and sulphur,
$R^{6a}$, $R^{6k}$ and $R^{6b}$ are Independently selected from $C_1$-$C_8$-alkyl, $C_1$-$C_8$-hydroxyalkyl, $C_1$-$C_8$-alkylamino($C_1$-$C_8$-alkyl), di ($C_1$-$C_8$alkyl)amlno($C_1$-$C_8$-alkyl), $C_1$-$C_6$-cyanoalkyl, a $C_3$-$C_{15}$-carbocydic group, $C_1$-$C_6$-haloalkyl and a 4- to 10-membered heterocyclic group having one or more heteroatoms selected from the group consisting of oxygen, nitrogen and sulphur;
$R^{6c}$, $R^{8d}$, $R^{6e}$ and $R^{6f}$ are independently H, $C_1$-$C_8$-alkyl, $C_1$-$C_8$-hydroxyalkyl, $C_1$-$C_8$-alkylamino($C_1$-$C_8$-alkyl), di ($C_1$-$C_8$-alky)amino($C_1$-$C_8$-alkyl), $C_1$-$C_8$-cyanoalkyl, a $C_3$-$C_{15}$-carbocyclic group, $C_1$-$C_8$-haloalkyl, a 4- to 10-membered heterocyclic group having one or more heteroatoms selected from the group consisting of oxygen, nitrogen and sulphur, or together with the nitrogen atom to which they are attached, form a $C_4$-$C_{10}$-heterocyclic group;
$R^{6g}$ and $R^{6i}$ are independently selected from H, $C_1$-$C_8$-alkyl, $C_1$-$C_8$-hydroxyalkyl, $C_1$-$C_8$-alkylamino($C_1$-$C_8$-alkyl), di ($C_1$-$C_8$-alkyl)amino($C_1$-$C_8$-alkyl), $C_1$-$C_8$-cyanoalkyl, a $C_3$-$C_{15}$-carbocyclic group, $C_1$-$C_8$-haloalkyl and a 4- to 10-membered heterocyclic group having one or more heteroatoms selected from the group consisting of oxygen, nitrogen and sulphur,
$R^{6h}$ and $R^{6j}$ are independently selected from $C_1$-$C_8$-alkyl, a $C_3$-$C_{15}$-carbocyclic group, a 4-to 10-membered heterocyclic group having one or more heteroatoms selected from the group consisting of oxygen, nitrogen and sulphur, $C_1$-$C_8$-hydroxyalkyl, amino($C_1$-$C_8$-alkyl), $C_1$-$C_8$-alkylamino($C_1$-$C_8$-alkyl), di($C_1$-$C_8$-alkyl)amino($C_1$-$C_8$-alkyl) and $C_1$-$C_6$-cyanoalkyl;
W is selected from $C_3$-$C_{15}$-carbocyclic group and 4- to 10-membered heterocycle having one or more heteroatoms selected from the group consisting of oxygen, nitrogen and sulphur;
m is an integer selected from 1-3; and
n is an integer selected from 1-4.

in formula (I), the following meanings are preferred independently, collectively or in any combination:

(i) $R^1$ and $R^2$ are independently H or $C_1$-$C_8$-alkyl;

(ii) $R^3$ and $R^4$ are H or $C_1$-$C_8$-alkyl;
(iii) $R^5$ is cyano;
(iv) $R^8$ is H, halogen, $C_1$-$C_8$-alkyl, $C_1$-$C_8$-haloalkyl, cyano, $SO_2R^{6a}$ or $-SR^{6b}$;
(v) $R^{6a}$ and $R^{6b}$ are independently selected from $C_1$-$C_8$-alkyl, a $C_3$-$C_{15}$-carbocyclic group and $C_1$-$C_8$-haloalkyl;
(vi) W is a $C_3$-$C_{10}$-carbocyclic group or a 4- to 10-membered heterocyclic group having one or more ring oxygen atoms;
(vii) m is 1; and
(viii) n is an integer selected from 1-4.

Preferred compounds of formula (I), in free or pharmaceutically acceptable salt form, include those of formula (Ia)

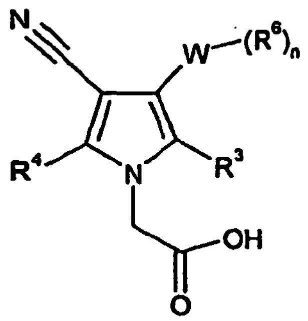

(Ia)

where $R^3$, $R^4$, $R^6$ and n are as hereinbefore defined.

More preferred compounds of formula (I), in free or pharmaceutically acceptable salt form, include those of formula (Ia)
wherein
$R^3$ and $R^4$ are H;
$R^6$ is selected from H, halogen, haloalkyl, cyano, $SO_2R^{6a}$ and $-SR^{6b}$;
$R^{6a}$ and $R^{6b}$ are independently selected from $C_1$-$C_4$-alkyl, $C_3$-$C_{10}$-carbocyclic group and $C_1$-$C_4$-haloalkyl;
W is a $C_6$-$C_{10}$-aromatic carbocyclic group or a 6- to 10-membered heterocyclic group having an aromatic ring and one or more ring oxygen atoms; and
n is an integer selected from 2-3.

In another embodiment, the present invention provides for the use of a compound of formula (I) in any of the aforementioned embodiments, in free or pharmaceutically acceptable salt form, for the manufacture of a medicament for the treatment of an inflammatory or allergic condition, particularly an inflammatory or obstructive airways disease.

WO 03101981 and WO 2005116001 disclose CRTh2 antagonists. Some 4-CN pyrrole derivatives are disclosed in PEST MANAGEMENT SCIENCE, 60, 2004, 1063.

***Definitions***

Terms used in the specification have the following meanings:

"Optionally substituted", as used herein, means the group referred to can be substituted at one or more positions by any one or any combination of the radicals listed thereafter.

"Halogen" or "halo" may be fluorine, chlorine, bromine or iodine; preferably it is bromine or chlorine or fluorine.

"$C_1$-$C_8$-Alkyl" denotes straight-chain or branched $C_1$-$C_8$-alkyl, which may be, e.g., methyl, ethyl, n-propyl, isopropyl, n-butyl, isobutyl, sec-butyl, tert-butyl, straight- or branched-pentyl, straight- or branched-hexyl, straight- or branched-heptyl or straight- or branched-octyl. Preferably, $C_1$-$C_8$-alkyl is $C_1$-$C_4$-alkyl.

"$C_3$-$C_{15}$-Carbocyclic group", as used herein, denotes a carbocyclic group having 3- to 15-ring carbon atoms, e.g., a monocyclic group, either cycloaliphatic, such as a $C_3$-$C_8$-cycloalkyl, e.g., cyclopropyl, cyclobutyl, cyclopentyl, cyclohexyl, cycloheptyl or cyclooctyl; or aromatic, such as phenyl, phenylene, benzenetriyl, naphthyl, naphthylene or naphthalenetriyl; or a bicyclic group, such as bicyclooctyl, bicyclononyl including indanyl and indenyl, and bicyclodecyl including naphthyl. Preferably, the $C_3$-$C_{15}$-carbocyclic group is a $C_3$-$C_{10}$-carbocyclic group, particularly a $C_6$-$C_{10}$-aromatic carbocyclic group, e.g., phenyl, phenylene, benzenetriyl, naphthyl, naphthylene or naphthalenetriyl

group. The $C_3$-$C_{15}$-carbocyclic group can be substituted with 1-3 substituents or unsubstituted. Preferred substituents include halo, cyano, amino, nitro, carboxy, $C_1$-$C_8$-alkyl, $C_1$-$C_8$-haloalkyl, $C_1$-$C_8$-alkoxy, $C_1$-$C_8$-cyanoalkyl, $C_1$-$C_8$-alkylcarbonyl, $C_1$-$C_8$-alkoxycarbonyl, $C_1$-$C_8$-haloalkoxy, carboxy-$C_1$-$C_8$-alkyl, $C_1$-$C_8$-alkylamino, di($C_1$-$C_8$-alkylamino), $C_1$-$C_8$-alkylsulfonyl, -$SO_2NH_2$ ($C_1$-$C_8$-alkylamino)sulfonyl, di($C_1$-$C_8$-alkyl)aminosulfonyl, aminocarbonyl, $C_1$-$C_8$-alkylaminocarbonyl and di($C_1$-$C_8$-alkyl)aminocarbonyl, a $C_3$-$C_{10}$-carbocyclic group and a 5- to 12-membered heterocyclic group having at least one ring heteroatom selected from nitrogen, oxygen and sulphur.

"$C_6$-$C_{15}$-Aromatic carbocyclic group", as used herein, denotes a divalent aromatic group having 6- to 15-ring carbon atoms, e.g., phenylene, naphthylene or anthrylene. The $C_6$-$C_{15}$-aromatic group can be substituted with 1-3 substituents or can be unsubstituted. Preferred substituents include halo, cyano, amino, nitro, carboxy, $C_1$-$C_8$-alkyl, halo-$C_1$-$C_8$-alkyl, $C_1$-$C_8$-alkoxy, $C_1$-$C_8$-cyanoalkyl, $C_1$-$C_8$-alkylcarbonyl, $C_1$-$C_8$-alkoxycarbonyl; $C_1$-$C_8$-haloalkoxy, carboxy-$C_1$-$C_8$-alkyl, $C_1$-$C_8$-alkylamino, di($C_1$-$C_8$-alkylamino), $C_1$-$C_8$-alkylsulfonyl,-$SO_2NH_2$, ($C_1$-$C_8$-alkylamino)sulfonyl, di($C_1$-$C_8$-alkyl)aminosulfonyl, aminocarbonyl, $C_1$-$C_8$-alkylaminocarbonyl and di($C_1$-$C_8$-alkyl)aminocarbonyl, a $C_3$-$C_{15}$-carbocyclic group and a 5- to 12-membered heterocyclic group having at least one ring heteroatom selected from nitrogen, oxygen and sulphur.

"bivalent $C_3$-$C_8$-cycloaliphatic" denotes cycloalkylene having 3- to 8-ring carbon atoms, e.g., a monocyclic group, such as a cyclopropylene, cyclobutylene, cyclopentylene, cyclohexylene, cycloheptylene or cyclooctylene, any of which can be substituted by one or more, usually one or two, $C_1$-$C_4$-alkyl groups; or a bicyclic group, such as bicycloheptylene or bicyclooctylene. Preferably "$C_3$-$C_8$-cycloalkylene" is $C_3$-$C_5$-cycloalkylene, e.g., cyclopropylene, cyclobutylene or cyclopentylene.

"$C_1$-$C_8$-Alkoxy" denotes straight-chain or branched $C_1$-$C_8$-alkoxy which may be, e.g., methoxy, ethoxy, *n*-propoxy, isopropoxy, *n*-butoxy, isobutoxy, *sec* butoxy, *tert* butoxy, straight- or branched-pentoxy, straight- or branched-hexyloxy, straight- or branched-heptyloxy or straight- or branched-octyloxy. Preferably, $C_1$-$C_8$-alkoxy is $C_1$-$C_4$-alkoxy.

"$C_1$-$C_8$-Haloalkyl" and "$C_1$-$C_8$-haloalkoxy" denote $C_1$-$C_8$-alkyl and $C_1$-$C_8$-alkoxy, as hereinbefore defined, substituted by one or more halogen atoms, preferably one, two or three halogen atoms, preferably fluorine, bromine or chlorine atoms. Preferably, $C_1$-$C_8$-haloalkyl is $C_1$-$C_4$-alkyl substituted by one, two or three fluorine, bromine or chlorine atoms. Preferably, $C_1$-$C_8$-haloalkoxy is $C_1$-$C_4$-aikoxy substituted by one, two or three fluorine, bromine or chlorine atoms. "$C_1$-$C_8$-Hydroxyalkyl" denotes $C_1$-$C_8$-alkyl as hereinbefore defined, substituted by at least one hydroxy group.

"$C_1$-$C_8$-Cyanoalkyl" denotes $C_1$-$C_8$-alkyl, as hereinbefore defined, substituted by at least one cyano group.

"$C_1$-$C_8$-Alkylsulfonyl", as used herein, denotes $C_1$-$C_8$-alkyl, as hereinbefore defined, linked to -$SO_2$-. Preferably, $C_1$-$C_8$-alkylsulfonyl is $C_1$-$C_4$-alkylsulfonyl.

"$C_1$-$C_8$-Haloalkylsulfonyl", as used herein, denotes $C_1$-$C_8$-haloalkyl, as hereinbefore defined, linked to -$SO_2$-. Preferably. $C_1$-$C_8$-haloalkylsulfonyl is $C_1$-$C_4$-haloatkylsulfonyl, especially trifluoromethylsulfonyl.

"Amino-$C_1$-$C_8$-alkyl" and "amino-$C_1$-$C_8$-alkoxy" denote amino attached by a nitrogen atom to $C_1$-$C_8$-alkyl, e.g., $NH_2$-($C_1$-$C_8$)-, or to $C_1$-$C_8$-alkoxy, e.g., $NH_2$-($C_1$-$C_8$)-O-, respectively, as hereinbefore defined. Preferably, amino-$C_1$-$C_8$-alkyl and amino-$C_1$-$C_8$-alkoxy are, respectively, amino-$C_1$-$C_4$-alkyl and amino-$C_1$-$C_4$-alkoxy.

"$C_1$-$C_8$-Alkylamino" and "di($C_1$-$C_8$-alkyl)amino" denote amino substituted respectively by one or two $C_1$-$C_8$-alkyl groups, as hereinbefore defined, which may be the same or different. Preferably, $C_1$-$C_8$-alkylamino and di ($C_1$-$C_6$-alkyl)amino are respectively $C_1$-$C_4$-alkylamino and di($C_1$-$C_4$-alkynamino.

"$C_1$-$C_8$-Alkyl amino-$C_1$-$C_8$-alkyl" and "di($C_1$-$C_8$-alkyl)amino $C_1$-$C_8$-alkyl" denote $C_1$-$C_8$-alkyl, as hereinbefore defined, substituted respectively by $C_1$-$C_8$-alkylamino or di($C_1$-$C_8$-alkyl)amino, as hereinbefore defined. Preferably, $C_1$-$C_8$-alkylamino-$C_1$-$C_8$-alkyl and di($C_1$-$C_8$-alkyl)amino-$C_1$-$C_8$-alkyl are, respectively, $C_1$-$C_4$-alkylamino-$C_1$-$C_4$-alkyl and di($C_1$-$C_4$-alkyl)amino-$C_1$-$C_4$-alkyl.

"Amino-(hydroxy)-$C_1$-$C_8$-alkyl" denotes amino attached by a nitrogen atom to $C_1$-$C_8$-alky) and hydroxy attached by an oxygen atom to the same $C_1$-$C_8$-alkyl. Preferably, amino-(hydroxy)-$C_1$-$C_8$-alkyl is amino-(hydroxy)-$C_2$-$C_4$-alkyl.

"Carboxy-$C_1$-$C_8$-alkyl" and "carboxy-$C_1$-$C_8$-alkoxy" denote carboxy attached by a carbon atom to $C_1$-$C_8$-alkyl or

$C_1$-$C_8$-alkoxy, respectively, as hereinbefore defined. Preferably, carboxy-$C_1$-$C_8$-alkyl and carboxy-$C_1$-$C_8$-alkoxy are, respectively, carboxy-$C_1$-$C_4$-alkyl and carboxy-$C_1$-$C_4$-alkoxy.

"$C_1$-$C_8$-Alkylcarbonyl", "$C_1$-$C_8$-alkoxycarbonyl" and "$C_1$-$C_8$-haloalkylcarbonyl" denote $C_1$-$C_8$-alkyl, $C_1$-$C_8$-alkoxy or $C_1$-$C_8$-haloalkyl, respectively, as hereinbefore defined, attached by a carbon atom to a carbonyl group. "$C_1$-$C_8$-Alkoxycarbonyl" denotes $C_1$-$C_8$-alkoxy, as hereinbefore defined, wherein the oxygen of the alkoxy group is attached to the carbonyl carbon. Preferably, $C_1$-$C_8$-alkylcarbonyl, $C_1$-$C_8$-alkoxycarbonyl and $C_1$-$C_8$-haloalkylcarbonyl are, respectively, $C_1$-$C_4$-alkylcarbonyl, $C_1$-$C_4$-alkoxycarbonyl and $C_1$-$C_4$-haloalkylcarbonyl.

"$C_1$-$C_8$-Alkylamino" and "di($C_1$-$C_8$-alkyl)amino" denote $C_1$-$C_8$-alkyl, as hereinbefore defined, attached by a carbon atom to an amino group. The $C_1$-$C_8$-alkyl groups in di($C_1$-$C_8$-alkyl)amino may be the same or different Preferably, $C_1$-$C_8$-alkylamino and di($C_1$-$C_8$-alkyl)amino are, respectively, $C_1$-$C_4$-alkylamino and di($C_1$-$C_4$-alkyl)amino.

"$C_1$-$C_8$-Alkylaminocarbonyl" and "di($C_1$-$C_8$-alkyl)aminocarbonyl" denote $C_1$-$C_8$-alkylamino and di($C_1$-$C_8$-alkyl)amino, respectively, as hereinbefore defined, attached by a nitrogen atom to the carbon atom of a carbonyl group. Preferably, $C_1$-$C_8$-alkylaminocarbonyl and di($C_1$-$C_8$-alkyl)-aminocarbonyl are, respectively, $C_1$-$C_4$-alkylaminocarbonyl and di($C_1$-$C_4$-alkyl)-aminocarbonyl.

"Four (4)- to 10-membered heterocyclic group containing at least one ring heteroatom selected from the group consisting of nitrogen, oxygen and sulphur", as used herein, may be monocyclic or bicyclic, e.g., furan, tetrahydrofuran, pyrrole, pyrrolidine, pyrazole, imidazole, triazole, isotriazole, tetrazole, thiadiazole, isothiazole, oxadiazole, pyridine, oxazole, isoxazole, pyrazine, pyridazine, pyrimidine, piperidine, piperazine, morpholine, triazine, oxazine, thiazole, quinoline, isoquinoline, benzothiophene, benzoxazole, benzisoxazole, benzothiazole, benzisothiazole, benzofuran, indole, indazole benzodioxole or benzimidazole.

Preferred heterocyclic groups include piperazine, morpholine, imidazole, isotriazole, pyrazole, pyridine, furan, oxazole, oxadiazole, isoxazole, thiazole, tetrazole benzothiophene, benzoxazole, benzothiazole, benzodioxole and benzofuran. The 4- to 10-membered heterocyclic group can be unsubstituted or substituted. Preferred substituents include halo, cyano, oxo, hydroxy, carboxy, nitro, $C_1$-$C_8$-alkyl, $C_1$-$C_8$-cyanoalkyl, $C_1$-$C_8$-alkylcarbonyl, hydroxy-$C_1$-$C_8$-alkyl, $C_1$-$C_8$-haloalkyl, amino-$C_1$-$C_8$-alkyl, amino(hydroxy)$C_1$-$C_8$-alkyl and $C_1$-$C_8$-alkoxy optionally substituted by aminocarbonyl. Especially preferred substituents include halo, oxo, $C_1$-$C_4$-alkyl, $C_1$-$C_4$-alkylcarbonyl, hydroxy-$C_1$-$C_4$-alkyl, $C_1$-$C_4$-haloalkyl, amino-$C_1$-$C_4$-alkyl and amino(hydroxy)$C_1$-$C_4$-alkyl.

Throughout this specification and in the claims that follow, unless the context requires otherwise, the word "comprise", or variations, such as "comprises" or "comprising", will be understood to imply the inclusion of a stated integer or step or group of integers or steps but not the exclusion of any other integer or step or group of integers or steps.

Many of the compounds represented by formula (I) are capable of forming acid addition salts, particularly pharmaceutically acceptable acid addition salts. Pharmaceutically acceptable acid addition salts of the compound of formula (I) include those of inorganic acids, e.g., hydrohalic acids, such as hydrochloric acid or hydrobromic acid; nitric acid; sulphuric acid; phosphoric acid; and organic acids, e.g., aliphatic monocarboxylic acids, such as formic acid, acetic acid, diphenylacetic acid, triphenylacetic acid, caprylic acid, dichloroacetic acid, trifluoroacetic acid, hippuric acid, propionic acid and butyric acid; aliphatic hydroxy acids, such as lactic acid, citric acid, gluconic acid, mandelic acid, tartaric acid or malic acid; dicarboxylic acids, such as adipic acid, aspartic acid, fumaric acid, glutamic acid, maleic acid, malonic acid, sebacic acid or succinic acid; aromatic carboxylic acids, such as benzoic acid, *p*-chlorobenzoic acid, or nicotinic acid; aromatic hydroxy acids, such as o-hydroxybenzoic acid, *p*-hydroxybenzoic acid, 1-hydroxynaphthalene-2-carboxylic acid or 3-hydroxynaphthalene-2-carboxylic acid; and sulfonic acids, such as ethanesulfonic acid, ethane-1,2-disulfonic acid, 2-hydroxyethanesulfonic acid, methanesulfonic acid, (+)-camphor-10-sulfonic acid, benzenesulfonic acid, naphthalene-2-sulfonic acid, naphthalene-1,5-disulfonic acid or *p*-toluenesulfonic acid. These salts may be prepared from compounds of formula (I) by known salt-forming procedures.

Compounds of formula (I) contain acidic, e.g., carboxyl, groups, and are also capable of forming salts with bases, in particular, pharmaceutically acceptable bases, such as those well-known in the art; suitable such salts include metal salts, particularly, alkali metal or alkaline earth metal salts, such as sodium, potassium, magnesium, calcium or zinc salts; or salts with ammonia or pharmaceutically acceptable organic amines or heterocyclic bases, such as benethamine, benzathine, diethanolamine, ethanolamine, 4(2-hydroxyethyl)morpholine,1-(2-hydroxyethy)pyrrolidine, *N*-methyl glucamine, piperazine, triethanolamine or tromethamine. These salts may be prepared from compounds of formula (I) by known salt-forming procedures.

In those compounds where there is an asymmetric carbon atom or an axis of chirality the compounds exist in individual optically active isomeric forms or as mixtures thereof, e.g., as racemic or diastereomeric mixtures. The present invention embraces both individual optically active R and S isomers, as well as mixtures, e.g., racemic or diastereomeric

mixtures thereof.

Specific especially preferred compounds of formula (I) include those hereinafter described in the Examples:

Since prodrugs are known to enhance numerous desirable qualities of pharmaceuticals, e.g., solubility, bioavailability, manufacturing, etc., the compounds of the present invention may be delivered in prodrug form. Thus, the present invention relates to also prodrugs of the presently claimed compounds, methods of delivering the same and compositions containing the same. "Prodrugs" are intended to include any covalently bonded carriers which release an active parent drug of the present invention *in vivo* when such prodrug is administered to a mammalian subject. Prodrugs of the present invention are prepared by modifying functional groups present in the compound in such a way that the modifications are cleaved, either in routine manipulation or *in vivo,* to the parent compound. Prodrugs include compounds of the present invention wherein a carboxy, hydroxy, amino or sulfhydryl-group is bonded to any group that, when the prodrug of the present invention is administered to a mammalian subject, it cleaves to form a free carboxy, free hydroxyl, free amino or free sulfhydryl group, respectively. Examples of prodrugs include, but are not limited to, ester derivatives of carboxy functional groups, acetate, formate and benzoate derivatives of alcohol and amine functional groups in the compounds of the present invention.

"Therapeutically effective amount" is intended to include an amount of a compound of the present invention alone or an amount of the combination of compounds claimed or an amount of a compound of the present invention in combination with other active ingredients effective to treat the inflammatory diseases described herein.

As used herein, "treating" or "treatment" cover the treatment of a disease-state in a mammal, particularly in a human, and include:

(a) preventing the disease-state from occurring in a mammal, in particular, when such mammal is predisposed to the disease-state but has not yet been diagnosed as having it;
(b) inhibiting the disease-state, i.e., arresting its development; and/or
(c) relieving the disease-state, i.e., causing regression of the disease state.

*Synthesis*

Another embodiment of the present invention provides a process for the preparation of compounds of formula (I), in free or pharmaceutically acceptable salt form, which comprises the steps of:

(i) cleaving an ester group -$COOR^7$ in a compound of formula (II)

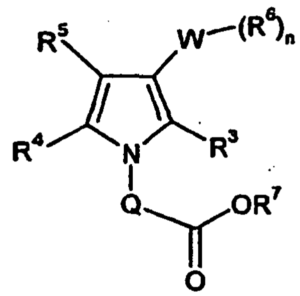

**(II)**

wherein
$R^7$ is $C_1$-$C_8$-alkyl; and
everything else as hereinbefore defined; and

(ii) recovering the resultant compound of formula (I), in free or pharmaceutically acceptable salt form.

The process may be carried out using known procedures for ester cleavage or analogously as hereinafter described in the Examples.

Starting materials for the process, and compounds for the preparation of those starting materials, may be novel or known; they may be prepared in accordance with known procedures or analogously, as hereinafter described in the Examples.

The present invention discloses compounds of formula (II)

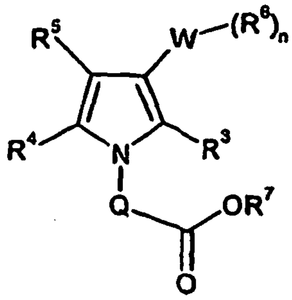

(II)

,

in free or pharmaceutically acceptable salt form,
wherein
Q is

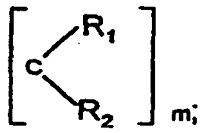

$R^1$ and $R^2$ are independently H, $C_1$-$C_8$-alkyl, or together with the carbon atom to which they are attached form a divalent $C_3$-$C_8$-cycloaliphatic group;
$R^3$ and $R^4$ are independently selected from H and $C_1$-$C_8$-alkyl;
$R^5$ is selected from H, halogen, $C_1$-$C_8$-alkyl, $C_1$-$C_8$-haloalkyl, a $C_3$-$C_{15}$-carbocyclic group, nitro, cyano, $SO_2R^{5a}$, $SOR^{5b}$, $SR^{5c}$, $C_1$-$C_8$-alkylcarbonyl, $C_1$-$C_8$-alkoxycarbonyl, $C_1$-$C_8$-alkoxy, $C_1$-$C_8$-haloalkoxy, carboxy, carboxy-$C_1$-$C_8$-alkyl, amino, amino($C_1$-$C_8$-alkyl), $C_1$-$C_8$-alkylamino, di($C_1$-$C_8$-alkyl)amino, $SO_2NR^{5d}R^{5e}$, -C(O)$NR^{5f}R^{5g}$, a $C_6$-$C_{15}$-aromatic carbocyclic group, and a 4- to 10-membered heterocyclic group having one or more heteroatoms selected from the group consisting of oxygen, nitrogen and sulphur,
$R^{5a}$, $R^{5b}$ and $R^{5c}$ are independently selected from $C_1$-$C_8$-alkyl, $C_1$-$C_8$-hydroxyalkyl, $C_1C_8$-alkylamino($C_1$-$C_8$-alkyl), di ($C_1$-$C_8$-alkyl)amino($C_1$-$C_8$-alkyl), $C_1$-$C_8$-cyanoalkyl, a $C_3$-$C_{15}$-carbocyclic group, $C_1$-$C_8$-haloalkyl and a 4- to 10-membered heterocyclic group having one or more heteroatoms selected from the group consisting of oxygen, nitrogen and sulphur;
$R^{5d}$, $R^{5e}$, $R^{5f}$ and $R^{5g}$ are independently H, $C_1$-$C_8$-alkyl, $C_1$-$C_8$-hydroxyalkyl, $C_1$-$C_8$-alkylamino($C_1$-$C_8$-alkyl), di ($C_1$-$C_8$-alkyl)amino($C_1$-$C_8$-alkyl), $C_1$-$C_8$-cyanoalkyl, a $C_3$-$C_{15}$-carbocyclic group, $C_1$-$C_8$-haloalkyl, a 4- to 10-membered heterocyclic group having one or more heteroatoms selected from the group consisting of oxygen, nitrogen and sulphur, or together with the nitrogen atom to which they are attached, form a $C_4$-$C_{10}$-heterocyclic group;
$R^6$ is selected from H, halogen, $C_1$-$C_8$-alkyl, $C_1$-$C_8$-haloalkyl, a $C_3$-$C_{15}$-carbocyclic group, nitro, cyano, $SO_2R^{6a}$, $C_1$-$C_8$-alkylcarbonyl, $C_1$-$C_8$-alkoxycarbonyl, $C_1$-$C_8$-alkoxy, $C_1$-$C_8$-haloalkoxy, -$SR^{6b}$, carboxy, carboxy-$C_1$-$C_8$-alkyl, amino, amino($C_1$-$C_8$-alkyl), $C_1$-$C_8$-alkylamino($C_1$-$C_8$-alkyl), di($C_1$-$C_8$-alkyl)amino($C_1$-$C_8$-alkyl), $C_1$-$C_8$-alkylamino, di ($C_1$-$C_8$-alkyl)amino, $SO_2NR^{6c}R^{6d}$, -C(O)$NR^{6e}R^{6f}$, $C_1$-$C_8$-hydroxyalkyl, $NR^{6g}SO_2R^{6h}$, $NR^{6i}$(CO)$R^{6j}$, $SOR^{6k}$, a $C_6$-$C_{15}$-aromatic carbocyclic group and a 4- to 10-membered heterocyclic group having one or more heteroatoms selected from the group consisting of oxygen, nitrogen and sulphur,
$R^{6a}$, $R^{6k}$ and $R^{8b}$ are independently selected from $C_1$-$C_8$-alkyl, $C_1$-$C_8$-hydroxyalkyl, $C_1$-$C_8$-alkylamino($C_1$-$C_8$-alkyl), di ($C_1$-$C_8$-alkyl)amino($C_1$-$C_8$-alkyl), $C_1$-$C_8$-cyanoalkyl, a $C_3$-$C_{15}$-carbocyclic group, $C_1$-$C_8$-haloalkyl and a 4- to 10-membered heterocyclic group having one or more heteroatoms selected from the group consisting of oxygen, nitrogen and sulphur,
$R^{6c}$, $R^{6d}$, $R^{6e}$ and $R^{6f}$ are independently H, $C_1$-$C_8$-alkyl, $C_1$-$C_8$-hydroxyalkyl, $C_1$-$C_8$-alkylamino($C_1$-$C_8$-alkyl), di ($C_1$-$C_8$-alkyl)amino($C_1$-$C_8$-alkyl), $C_1$-$C_8$-cyanoalkyl, a $C_3$-$C_{15}$-carbocyclic group, $C_1$-$C_8$-haloalkyl, a 4- to 10-membered heterocyclic group having one or more heteroatoms selected from the group consisting of oxygen, nitrogen and sulphur, or together with the nitrogen atom to which they are attached form a $C_4$-$C_{10}$-heterocyclic group;
$R^{6g}$ and $R^{6i}$ are independently selected from H, $C_1$-$C_8$-alkyl, $C_1$-$C_8$-hydroxyalkyl, $C_1$-$C_8$-alkylamino($C_1$-$C_8$-alkyl), di ($C_1$-$C_8$-alkyl)amino($C_1$-$C_8$-alkyl), $C_1$-$C_8$-cyanoalkyl, a $C_3$-$C_{15}$-carbocyclic group, $C_1$-$C_8$-haloalkyl and a 4- to 10-membered heterocyclic group having one or more heteroatoms selected from the group consisting of oxygen, nitrogen and sulphur,
$R^{6h}$ and $R^{6j}$ are independently selected from $C_1$-$C_8$-alkyl, a $C_3$-$C_{15}$-carbocyclic group, a 4-to 10-membered heterocyclic

group having one or more heteroatoms selected from the group consisting of oxygen, nitrogen and sulphur, $C_1$-$C_8$-hydroxyalkyl, amino($C_1$-$C_8$-alkyl), $C_1$-$C_8$-alkylamino($C_1$-$C_8$-alkyl), di($C_1$-$C_8$-alkyl)amino($C_1$-$C_6$-alkyl), and $C_1$-$C_8$-cyanoalkyl;

$R^7$ is $C_1$-$C_8$-alkyl;

W is selected from $C_6$-$C_{15}$-aromatic carbocyclic group and 5- to 10-membered heterocycle having one or more heteroatoms selected from the group consisting of oxygen, nitrogen and sulphur;

m is an integer selected from 1-3; and

n is an integer selected from 1-4.

Compounds of formula (II) may be used to prepare compounds of formula (I) in accordance with known procedures or analogously as hereinafter described in the Examples or Scheme 1.

Compounds of formula (II) may be prepared by reacting a compound of formula (III)

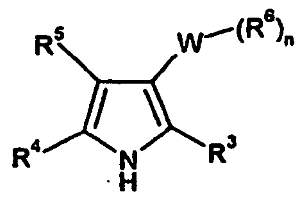

(III)

,

where all symbols are as hereinbefore defined, with a compound of formula (IV)

$$X\text{-}Q\text{-}COOR^7 \qquad (IV)$$

where

X is halogen; and

$R^7$ is as hereinbefore defined.

The reaction may be carried out using known procedures for reaction of amines with haloalkylcarboxylic esters, or analogously, as hereinafter described, in the Examples.

The compounds of formula (I) can be prepared, e.g., using the reactions and techniques described below. The reactions may be performed in a solvent appropriate to the reagents and materials employed and suitable for the transformations being effected. It will be understood by those skilled in the art of organic synthesis that the functionality present on the molecule should be consistent with the transformations proposed. This will sometimes require a judgment to modify the order of the synthetic steps or to select one particular process scheme over another in order to obtain a desired compound of the invention.

The various substituents on the synthetic intermediates and final products shown in the following reaction schemes can be present in their fully elaborated forms, with suitable protecting groups where required as understood by one skilled in the art, or in precursor forms which can later be elaborated into their final forms by methods familiar to one skilled in the art. The substituents can also be added at various stages throughout the synthetic sequence or after completion of the synthetic sequence. In many cases, commonly used functional group manipulations can be used to transform one intermediate into another intermediate, or one compound of formula (I) into another compound of formula (I). Examples of such manipulations are conversion of an ester or a ketone to an alcohol; conversion of an ester to a ketone; interconversions of esters, acids and amides; alkylation, acylation and sulfonylation of alcohols and amines; and many others. Substituents can also be added using common reactions, such as alkylation, acylation, halogenation or oxidation. Such manipulations are well-known in the art, and many reference works summarize procedures and methods for such manipulations. Some reference works which give examples and references to the primary literature of organic synthesis for many functional group manipulations, as well as other transformations commonly used in the art of organic synthesis are March's Organic Chemistry, 5th Edition, Wiley and Chichester, Eds. (2001); Comprehensive Organic Transformations, Larock, Ed., VCH (1989); Comprehensive Organic Functional Group Transformations, Katritzky et al., series editors, Pergamon (1995); Comprehensive Organic Synthesis, Trost and Fleming, series editors, Pergamon (1991); and Pavri and Trudell, J Orgr Chem, Vol. 62, No. 8, pp. 2649-2651 (1997).

Generally, compounds described in the scope of this patent application can be synthesized by the route described in Scheme **1**.

Scheme **1** depicts the general synthetic scheme when there is a nitrile substituent attached to either the 3- or 4-position of the pyrrole. For instance, in Scheme **1**, cinnamonitrile derivative **2** may be prepared by reaction of aldehyde derivative **1** in the presence of an inorganic base, such as sodium hydride, and a phosphonate derivative, preferably diethyl cyanomethylphosphonate in accordance with March, 5$^{th}$ ed., p. 1233. The cinnamonitrile derivative **2** may then

be reacted with an (arylsulfonyl)methylisocyanide, such as (*p*-toluenesulfonyl)methylisocyanide in the presence of a bass, as in Pavri and Trudell (1997), *supra,* to provide pyrrole derivative <u>**3**</u>. Pyrrole derivative <u>**3**</u> may be alkylated with an alkyl halide, such as methyl-2-bromoacetate, in the presence of a strong base, such as sodium hydride, to provide compound <u>**4**</u>. Compound <u>**4**</u> mary then be hydrolyzed to provide compound <u>**5**</u>.

**<u>Scheme 1</u>**

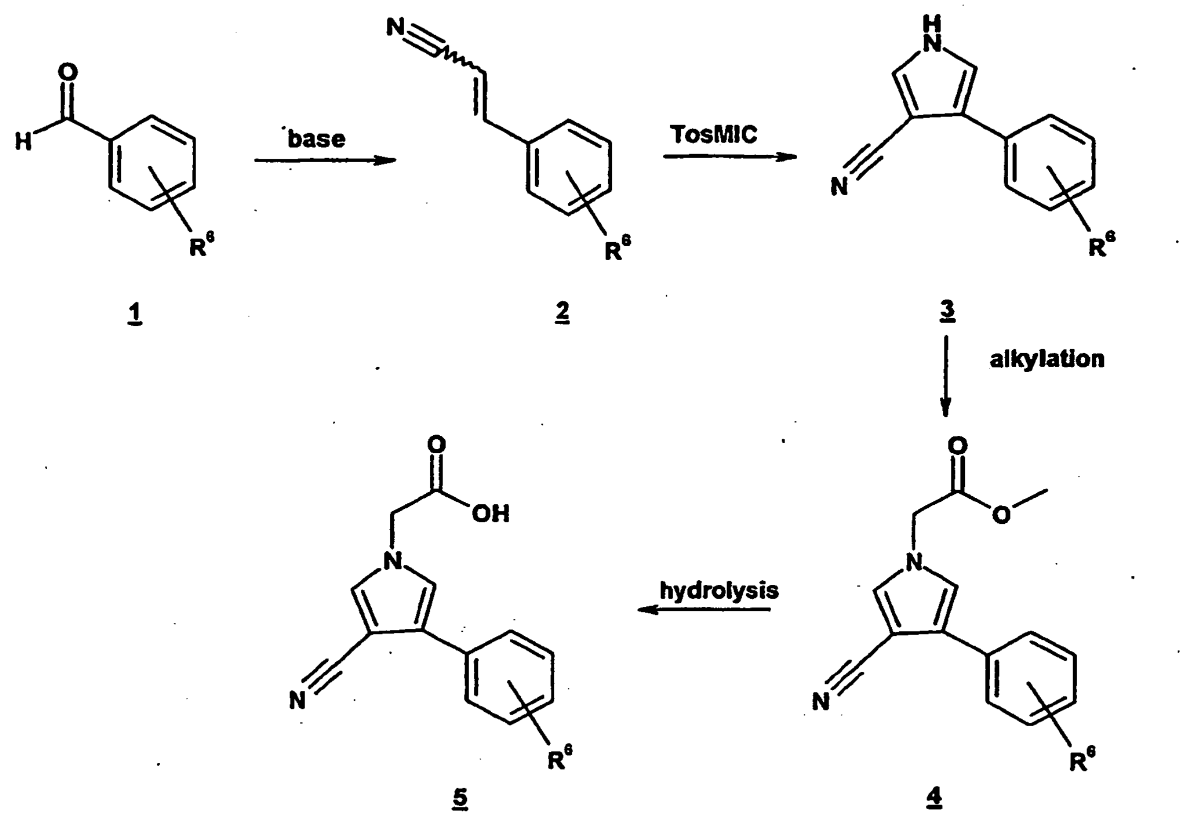

1 2 3 4 5

<u>The invention is illustrated by the following Examples.</u>

## <u>EXAMPLES</u>

### *General Conditions*

LCMS are recorded on an Agilent 1100 LC system with a Waters Xterra MS C18 4.6 x 100 5 μM column, eluting with 5-95% 10 mM aqueous ammonium bicarbonate in acetonitrile over 2.5 minutes, with negative ion electrospray ionization or 5-95% water + 0.1% TFA in acetonitrile with positive ion electrospray ionization. $[M+H]^+$ and $[M-H]^-$ refer to monoisotopic molecular weights.

***Abbreviations***

| | | | |
|---|---|---|---|
| AcOH | acetic acid | MeCN | acetonitrile |
| $BH_3$ | borane | MeOH | methanol |
| **$CuCl_2$** | copper (II) chloride | $MgSO_4$ | magnesium sulfate |
| DCM | dichloromethane | NaH | sodium hydride |
| DIBAL-H | di-*iso*-butylaluminium hydride | NaOH | sodium hydroxide |
| DMF | dimethylformamide | $Na_2SO_4$ | sodium sulfate |
| DMSO | dimethyl sulfoxide | PS-CDI | polymer supported |

(continued)

| | | | |
|---|---|---|---|
| $Et_3N$ | triethylamine | | carbodiimide |
| EtOAc | ethyl acetate | $SO_2$ | sulphur dioxide |
| HCl | hydrochloric acid | RT | room temperature |
| HOBt | 1-hydroxybenzotriazole | *t*-BuOK | potassium tert-butoxide |
| HPLC | high performance liquid chromatography | THF | tetrahydrofuran |
| UOH | lithium hydroxide | TosMIC | (*p*-toluenesulfonyl) methylisocyanide |

The following examples have been prepared using the process described herein.

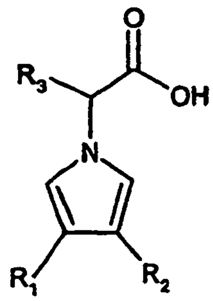

| $R_3$ = H, except in Example 67, where $R_3$ =$CH_3$ | | | |
|---|---|---|---|
| Example | $R_1$ | $R_2$ | [M+H]+/ [M-H]- |
| 1 | 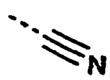 | 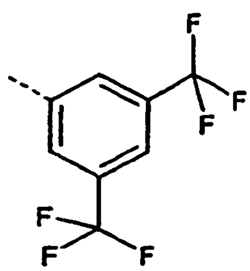 | 361 |
| 2 | 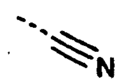 | 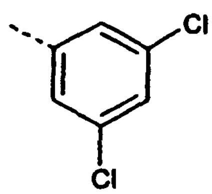 | 293/295 |
| 3 | 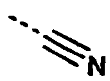 | 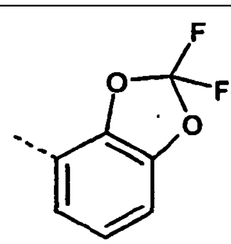 | 305 |

(continued)

| Example | $R_1$ | $R_2$ | [M+H]+/ $[M-H]^-$ |
|---|---|---|---|
| 4 | 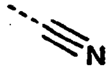 | 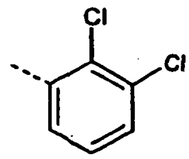 | 293 |
| 5 | 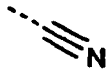 | 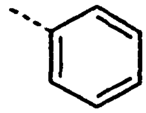 | 227 |
| 6 | 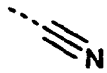 | 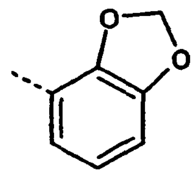 | 271 |
| 7 | 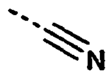 | 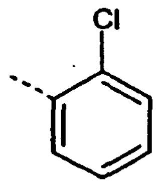 | 261 |
| 8 | 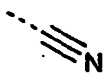 | 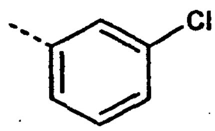 | 259/261 |
| 9 | 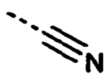 | 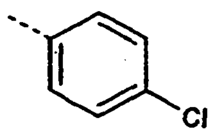 | 259 |
| 10 | 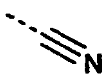 | 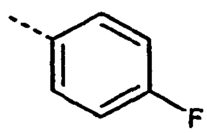 | 245 $[M+H]^+$ |
| 11 | 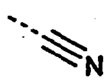 | 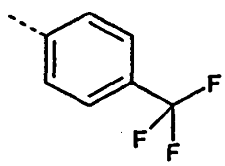 | 293 |
| 12 | 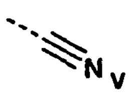 | 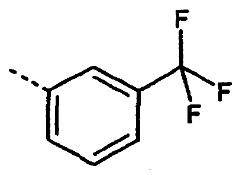 | 293 |
| 13 | 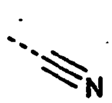 | 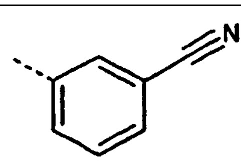 | 250 |

(continued)

| Example | $R_1$ | $R_2$ | [M+H]+/ [M-H]- |
|---|---|---|---|
| 14 | 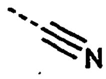 | 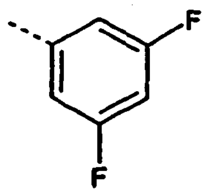 | 261 |
| 15 | 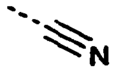 | 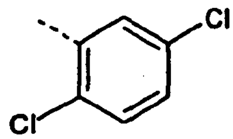 | 293/295 |
| 16 | 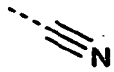 | 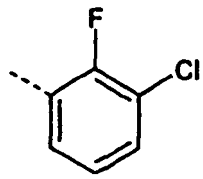 | 277 |
| 17 | 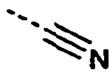 | 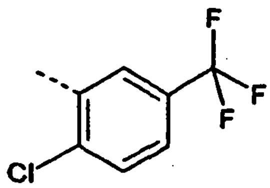 | 327 |
| 18 | 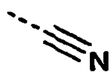 | 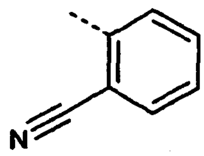 | 250 |
| 19 | 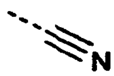 | 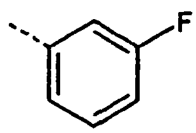 | 243 |
| 20 | 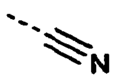 | 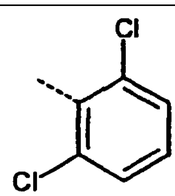 | 293/295 |
| 21 | 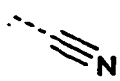 | 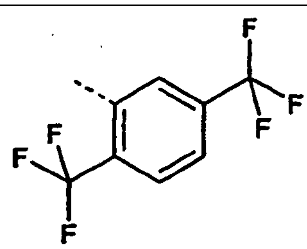 | 361 |

(continued)

| Example | $R_1$ | $R_2$ | $[M+H]^+$/ $[M-H]^-$ |
|---|---|---|---|
| 22 | 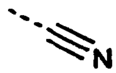 | 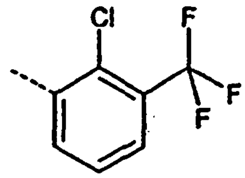 | 327 |
| 23 | 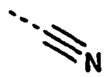 | 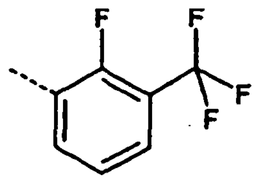 | 311 |
| 24 | 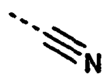 | 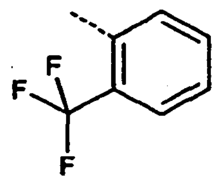 | 293 |
| 25 | 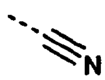 | 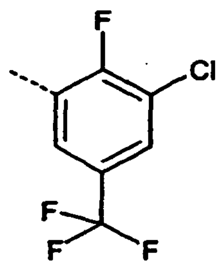 | 345/347 |
| 26 | 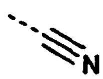 | 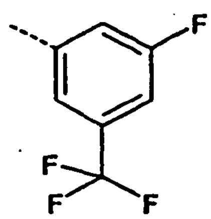 | 311 |
| 27 | 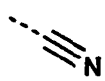 | 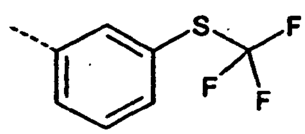 | 325 |
| 28 | 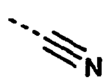 | 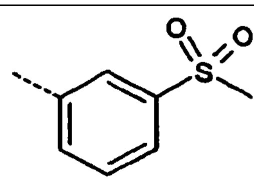 | 303 |
| 29 | 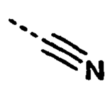 | 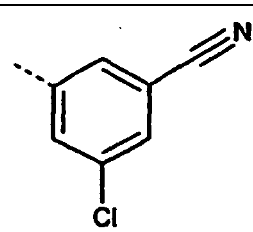 | 327 $(MH+CH_3CN)^+$ |

(continued)

| Example | $R_1$ | $R_2$ | [M+H]+/ [M-H]⁻ |
|---|---|---|---|
| 30 | 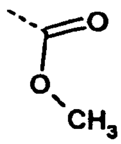 | 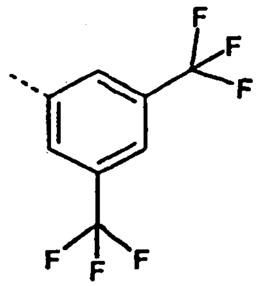 | 394 |
| 31 | 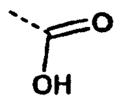 | 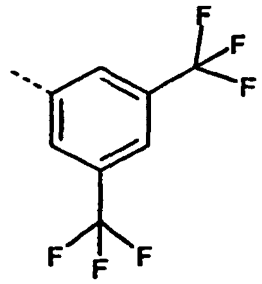 | 380 |
| 32 | 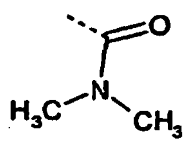 | 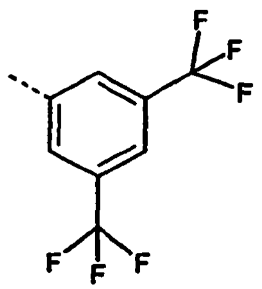 | 407 |
| 33 | 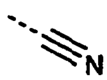 | 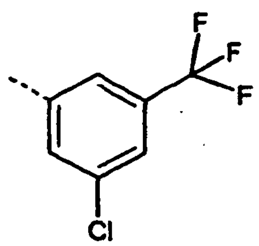 | 327/329 |
| 34 | 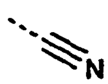 | 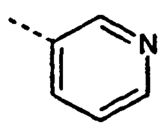 | 226 |
| 35 | 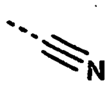 | 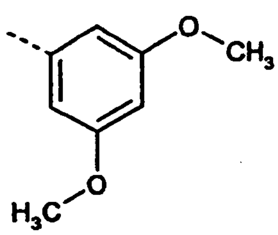 | 285 |

(continued)

| Example | $R_1$ | $R_2$ | [M+H]+/ [M-H]$^-$ |
|---|---|---|---|
| 36 | 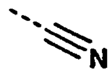 | 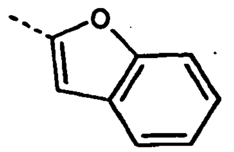 | 265 |
| 37 | 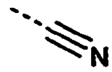 | 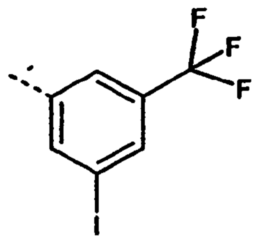 | 419 |
| 38 | 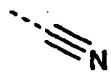 | 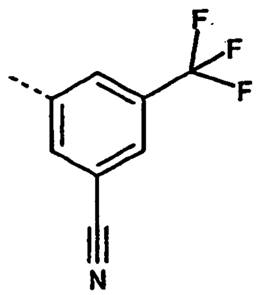 | 318 |
| 39 | 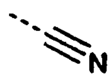 | 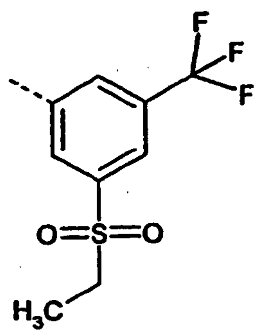 | 385 |
| 40 | 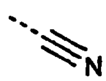 | 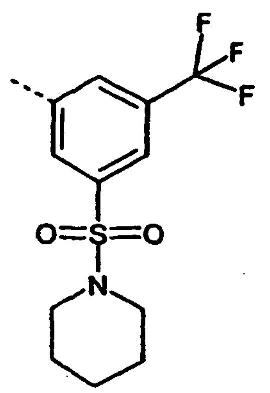 | 440 |

(continued)

| Example | $R_1$ | $R_2$ | $[M+H]^+$/ $[M-H]^-$ |
|---|---|---|---|
| 41 | 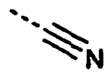 | 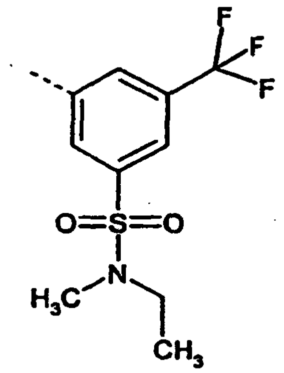 | 414 |
| 42 | 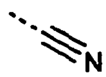 | 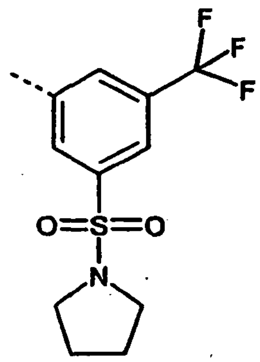 | 426 |
| 43 | 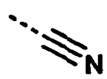 | 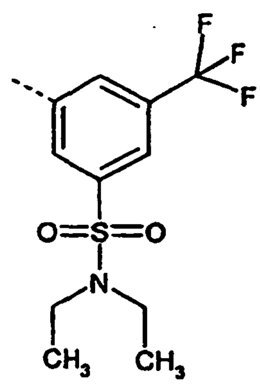 | 428 |
| 44 | 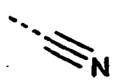 | 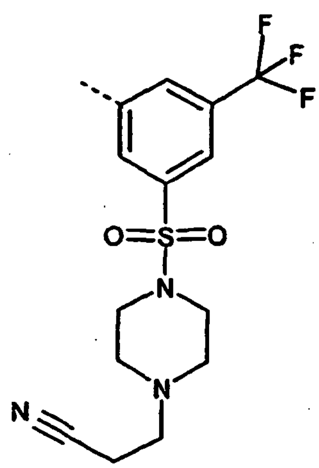 | 494 |

(continued)

| Example | $R_1$ | $R_2$ | [M+H]+/ [M-H]- |
|---|---|---|---|
| 45 | 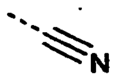 | 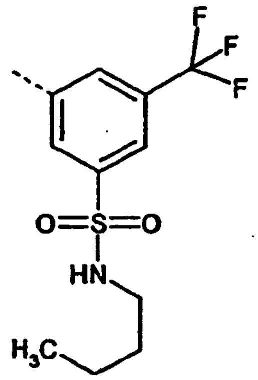 | 428 |
| 46 | 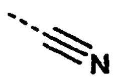 | 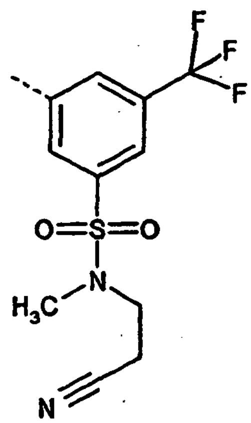 | 439 |
| 47 | 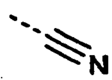 | 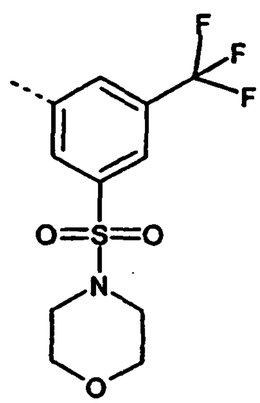 | 442 |
| 48 | 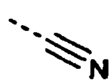 | 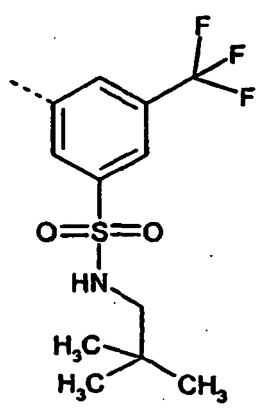 | 444 |

(continued)

| Example | $R_1$ | $R_2$ | [M+H]+/ [M-H]- |
|---|---|---|---|
| 49 | 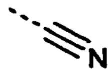 | 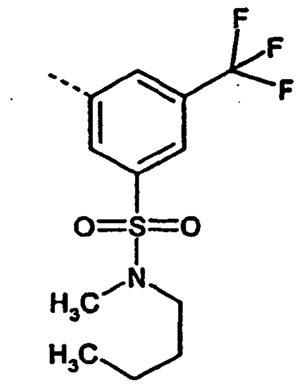 | 444 |
| 50 | 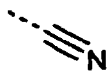 | 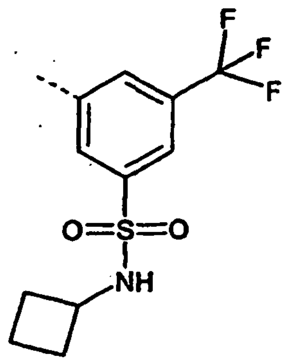 | 428 |
| 51 | 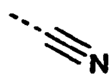 | 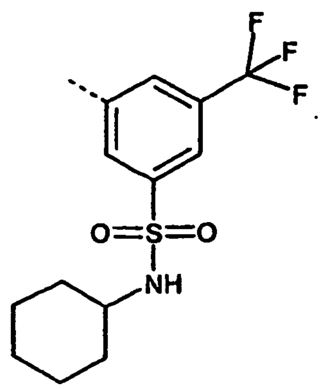 | 456 |
| 52 | 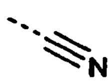 | 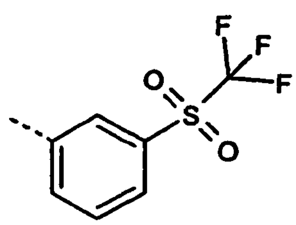 | 357 |

(continued)

| Example | $R_1$ | $R_2$ | [M+H]+/ [M-H]- |
|---|---|---|---|
| 53 | 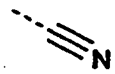 | 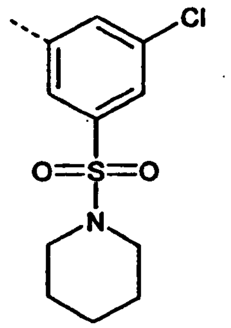 | 406 |
| 54 | 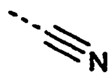 | 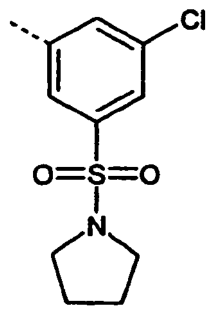 | 392 |
| 55 | 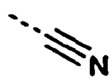 | 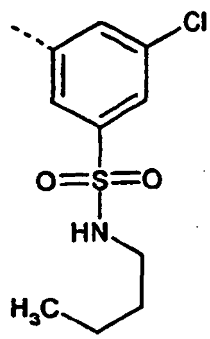 | 394 |
| 56 | 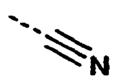 | 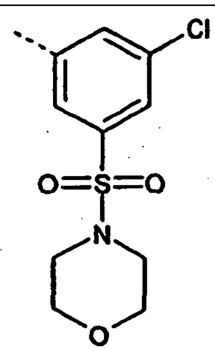 | 408 |

(continued)

| Example | $R_1$ | $R_2$ | [M+H]+/ [M-H]- |
|---|---|---|---|
| 57 | 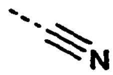 | 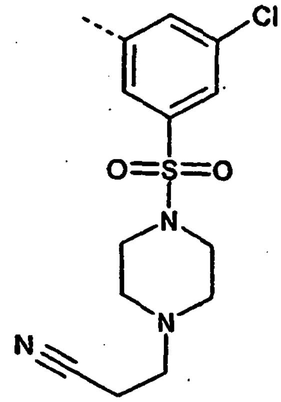 | 462 |
| 58 | 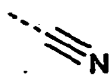 | 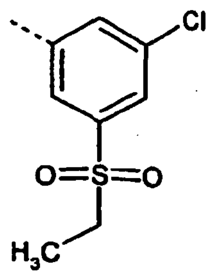 | 353/355 |
| 59 | 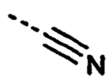 | 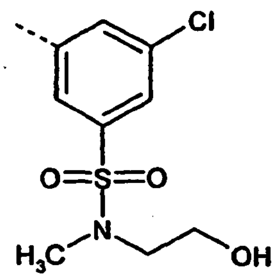 | 398 |
| 60 | 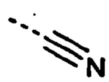 | 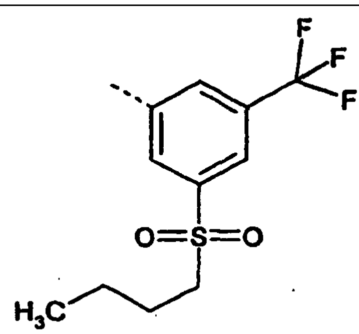 | 413 |
| 61 | 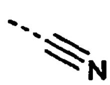 | 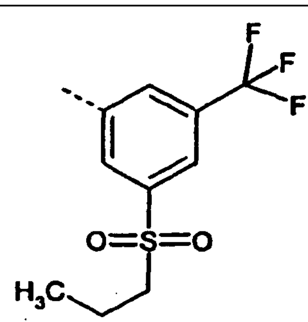 | 399 |

(continued)

| Example | $R_1$ | $R_2$ | [M+H]+/ [M-H]- |
|---|---|---|---|
| 62 | 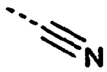 | 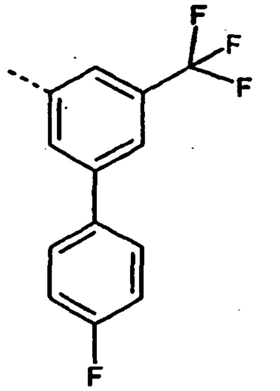 | 387 |
| 63 | 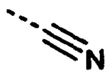 | 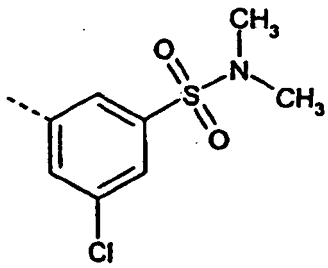 | 366 |
| 64 | 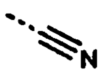 | 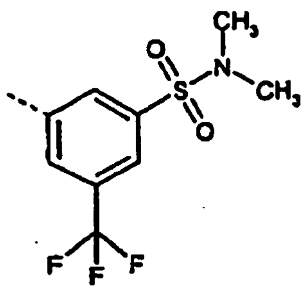 | 400 |
| **65** | 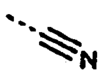 | 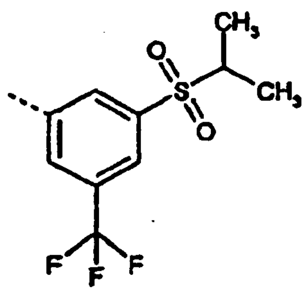 | 399 |
| 66 | 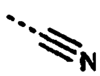 | 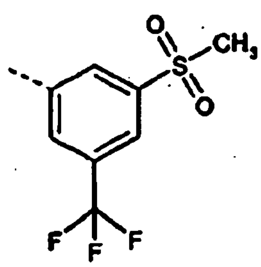 | 371 |

(continued)

| Example | $R_1$ | $R_2$ | $[M+H]^+$/ $[M-H]^-$ |
|---|---|---|---|
| 67 | 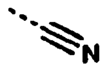 | 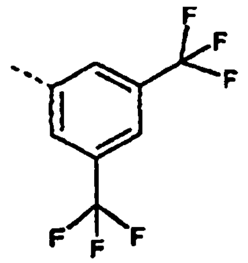 | 375 |
| Compounds 4, 30-23 are reference examples. | | | |

**Example 1**

**Preparatton of [3-(3,5-*bis*-trifluoromethyl-phenyl)-4-cyano-pyrrol-1-yl]-acetic acid**

a) To an ice-cooled stirring solution of *t*-BuOK (0.471 g, 4.2 mmol) in THF (20 mL) under inert atmosphere is added dropwise over 15-20 minutes a combined solution of commercially-available 3.5-*bis*(trifluoromethyl)cinnamonitrile (0.795 g, 3 mmol) and TosMIC (0.702g, 3.6 mmol) in THF (10 mL). One hour after the addition the ice bath is removed and the red-brown suspension is stirred at room temperature overnight The solvent is removed under reduced pressure and the residual solid is partitioned between EtOAc and water. The aqueous phase is extracted with EtOAc and the organic phases are combined, dried over $Na_2SO_4$ and the solvent is removed under reduced pressure to give a dark brown gum. The crude product is purified by flash chromatography (3:1 isohexane:EtOAc elution), to afford 4-(3,5-*bis*-trifluoromethyl-phenyl)-1*H*-pyrrole-3-carbonitrile; $[M+H]^+$ 305.

b) To an ice-cooled stirring suspension of NaH (60% dispersion in mineral oil; 19.7 mg, 0.494 mmol) in DMF (3 mL) is added under inert atmosphere a solution of 4-(3,5-*bis*-trifluoromethyl-phenyl)-1*H* pyrrole-3-carbonitrile (0.100 g, 0.329 mmol) in DMF. After 45 minutes, methyl-2-bromoacetate (37.4 μL, 0.395 mmol) is added and the reaction mixture is left to stir for 1.5 hours. The mixture is partitioned between water and EtOAc. The organic layer is washed with water and brine then dried over $MgSO_4$. The solvent is removed under reduced pressure to yield [3-(3,5-*bis*-trifluoromethyl-phenyl)-4-cyano-pyrrol-1-yl]-acetic acid methyl ester as a pale orange solid which is taken on without further characterisation to the next step.

c) 1 M Aqueous NaOH (80 μl, 0.08 mmol) is added dropwise to a solution of [3-(3,5-*bis*-trifluoromethyl-phenyl)-4-cyano-pyrrol-1-yl]-acetic acid methyl ester (30 mg, 0.08 mmol) in 1:1 THF:$H_2O$ (4 mL). After 2 hours, DCM is added and the mixture stirred for 10 minutes. The aqueous layer is isolated, acidified to pH 1 with 1 M HCl and extracted with DCM. The organic phase is evaporated to afford [3-(3,5-*bis*-trifluoromethyl-phenyl)-4-cyano-pyrrol-1-yl]-acetic acid as an off-white solid; $[M-H]^-$ 361.3.

**Example 2**

**Preparation of [3-cyano-4-(3,5-dichloro-phenyl)-pyrrol-1-yl]-acetic acid**

a) To an ice-cooled stirring suspension of NaH (60% dispersion in mineral oil; 0.23 g, 6 mmol) in dry THF (25 mL) under inert atmosphere is added dropwise diethyl (cyanomethyl)phosphonate (0.94 mL, 6 mmol). After 50 minutes at 0°C, a solution of 3,5-dichlorobenzaldehyde (0.875 g, 5 mmol) in dry THF (5 mL) is added over a 10 minutes period at 0°C. The reaction mixture is stirred at room temperature overnight. The solvent is removed under reduced pressure and the residue is partitioned between EtOAc and water. The aqueous phase is extracted with EtOAc and the organic phases are combined, washed with water, brine and dried over $MgSO_4$ and the solvent is removed under reduced pressure to give 3-(3,5-dtchioro-phenyl)-scrytonitrile as an orange solid which is taken to the next step without further characterization.

b) To an Ice-Cooled stirring solution of t-BuOK (0.673 g, 6 mmol) in THF (20 mL) under inert atmosphere is added dropwise over 20-30 minutes a combined solution of 3-(3,5-dichloro-phenyl)-acryionitrile (0.99 g, 5 mmol) and TosMIC (1.17 g, 6 mmol) in THF (10 mL). One hour after the addition the ice bath is removed and the reaction mixture is stirred at room temperature overnight. The solvent is removed under reduced pressure and the residual solid is partitioned between EtOAc and water. The aqueous phase is extracted with EtOAc and the organic phases are combined, washed with water, brine and dried over $MgSO_4$, and the solvent is removed under reduced pressure to give a brown solid. The crude product is purified by flash chromatography (gradient from iso-hexane to 1:1 isohexane:EtOAc), to afford 4-(3,5-dichloro-phenyl)-1*H*-pyrrole-3-carbonotrile; $[M.H]^-$ 2351237.

c) To an ice-cooled stirring suspension of NaH (60% dispersion in mineral oil; 24.3 mg, 0.633 mmol) In DMF (3 mL) is added dropwise and under inert atmosphere a solution of 4-(3,5-dichloro-phenyl)-1*H*-pyrrole-3-carbonitrile (0.100 g, 0.422 mmol) in DMF (2mL). After 45 minutes, methyl-2-bromoacetate (48 μL, 0.5(78 mmol) is added at 0˚C and the reaction mixture is left to stir at room temperature overnight. The mixture is partitioned between water and EtOAc. The organic layer is washed with water and brine then dried over $MgSO_4$. The solvent is removed under reduced pressure to yield [3-cyano-4-(3,5-dichloro-phenyl)-pyrrol-1-yl]-acetic acid methyl ester as orange solid which is taken on without further characterisation to the next step.

d) 1 M Aqueous UOH (194 μL, 0.194 mmol) is added dropwise at 0˚C to a solution of [3-cyano-4-(3,5-dichloro-phenyl)-pyrrol-1-yl]-acetic add methyl ester (60 mg, 0.194 mmol) in 1:1 THF:$H_2O$ (4 mL). The reaction mixture is stirred at 0˚C for 30 minutes then at room temperature overnight. DCM is added and the aqueous layer is isolated, acidified to pH 1 with 1 M HCl and extracted with DCM. The organic phase is evaporated to afford [3-cyano-4-(3,5-dichloro-phenyl)-pyrrol-1-yl]-acetic add as a solid; $[M-H]^-$ 293/295.

**Example 3-27**

These examples, namely, (3-Cyano-4-(2,2-difluoro-benzo[1,3]d)oxot-4-yl)-pyrrol-1-yl-acetic acid; reference example 4 -[3-Cyano-4-(2,3-dichloro-phenyl)-pyrrol-1-yl]-acetic acid, (3-cyano-4-phenyl-pyrrol-1.yl)acetic acid; (3-Benzo [1,3]dioxol-yl-4-cyano-pyrrol-1-yl-acetic acid: [3-(2-Chlorophenyl)-4-cyano-pyrrol-1-yl]-acetic acid; [3-(3-Chloro-phenyl)-4-cyano-pyrrol-1-yl]-acetic acid; [3-(4-Chloro-phenyl)-4-cyano-pyrrol-1-yl]-acetic acid; [3-Cyano-4(4-fluoro-phenyl)-pyrrol-1-yl]-acetic acid; [3-Cyano-4-(4-trifluoromethyl-phenyl)-pyrrol-1-yl]-acetic acid, [3-Cyano-4-(3-trifluoromethyl-phenyl)-pyrrol-1-yl)-acetic acid, [3-Cyano-4-(3-cyano-phenyl)-pyrrol-1-yl]-acetic acid, [3-Cyano-4-(3,5-difluoro-phenyl)-pyrrol-1-yl]-acetic acid, [3-Cyano-4-(2,5-dichloro-phenyl)-pyrrol-1-yl]-acetic acid, [3-(3-Chloro-2-fluoro-phenyl)-4-cyano-pyrrol-1-yl]-acetic acid, [3-(2-Chloro-5-trifluoromethyl-phenyl)-4-cyano-pyrrol-1-yl]-acetic acid, [3-Cyano-4-(2-cyano-phenyl)-pyrrol-1-yl]-acetic acid, [3-Cyano-4-(3-fluoro-phenyl)-pyrrol-1-yl]-acetic acid, [3-Cyano-4-(2,6-dichloro-phenyl)-pyrrol-1-yl]-acetic acid, [3-(2,5-Bis-trifluoromethylphenyl)4-cyano-pyrrol-1-yl]-acetic acid, [3-(2-Chloro-3-trifluoromethyl-phenyl)-4-cyano-pyrrol-1-yl]-acetic acid, [3-Cyano-4-(2-fluoro-3-trifluoromethyl-phenyl)-pyrrol-1-yl]-acetic acid, [3-Cyano-4-(2-trifluoromethyl-phenyn-pyrrol-1-yl]-acetic acid, [3-(3-Chloro-2-fluoro-5-trifluoromethyl-phenyl)-4-cyano-pyrrol-1-yl]-acetic acid, [3-Cyano-4-(3-fluoro-5-trifluoromethyl-phenyl)-pyrrol-1-yl]-acetic acid, and [3-Cyano-4-(3-trifluoromethylsulfanylphenyl)-pyrrol-1-yl]-acetic acid are prepared by similar processes as that described in Examples 1 and 2.

**Example 28**

**Preparation of [3-Cyano-4-(3-methanesulfonyl-phenyl)-pyrrol-1-yl]-acetic acid**

a) To an ice-cooled stirring solution of 1M $BH_3$ in THF (25 mL, 25 mmol) under inert atmosphere is added dropwise over a 10 minutes period a solution of 3-methanesulfonylbenzoic acid (2g, 10 mmol) in THF (20 mL). The reaction mixture is stirred at room temperature overnight then cooled down to 0˚C before adding water (2 mL). The solvent is removed under reduced pressure and the residue is partitioned between DCM and NaOH. The organic layer is isolated and dried over $MgSO_4$ and filtered. The solvent is removed under reduced pressure to give (3-methanesulfonyl-phenyl)-methanol; $[M-H]^-$ 185.

b) To a solution of oxalyl chloride (1.05 mL, 12 mmol) in DCM (15 mL) at -78˚C is added a solution of DMSO (1.9 mL, 26.6 mmol) in DCM (15 mL) over 5 minutes. The reaction mixture is stirred for 20 minutes, then a solution of (3-methanesulfonyl-phenyl)-methanol (0.992 g, 5.33 mmol) in DCM (20 mL) is added over a 5 minute period. The reaction mixture is stirred for 15 minutes then $Et_3N$ (3.78 mL, 27.2 mmol) is added over a 5 minute period. The ice

bath is removed and water (30 mL) is added at room temperature. The reaction mixture is stirred for 10 minutes, then the organic layer is isolated, dried over $MgSO_4$ and filtered. The solvent is removed under reduced pressure to yield 3-methanesulfonyl-benzaldehyde as a pale orange solid; $[M-H]^-$ 183.

c) To an ice-cooled stirring suspension of NaH (60% dispersion in mineral oil; 0.23 g, 6 mmol) in dry THF (25 mL) under inert atmosphere is added dropwise diethyl (cyanomethyl)phosphonate (0.94 mL, 6 mmol). After 50 minutes at 0˚C, a solution of 3-methanesulfonyl-benzaldehyde (0.918 g, 5 mmol) in dry THF (5 mL) is added dropwise over a 10 minutes period at 0˚C**.** The reaction mixture is stirred at room temperature overnight. The solvent is removed under reduced pressure and the residue is partitioned between EtOAc and water. The aqueous phase is extracted with EtOAc and the organic phases are combined, washed with water, brine and dried over $MgSO_4$ and the solvent is removed under reduced pressure to give 3-(3-methanesulfonylphenyl)-acrylonitrile as an orange sticky solid; $[M-H]^-$ 206.

d) To an ice-cooled stirring solution of *t*-BuOK (0.673 g, 6 mmol) in THF (20 mL) under inert atmosphere is added dropwise over 20-30 minutes a combined solution of 3-(3-methanesulfonylphenyl)-acrylonitrile (1 g, 5 mmol) and TosMIC (1.17 g, 6 mmol) in THF (10 mL). One hour after the addition the ice bath is removed and the reaction mixture is stirred at room temperature overnight. The solvent is removed under reduced pressure and the residue is partitioned between EtOAc and water. The aqueous phase is extracted with EtOAc and the organic phases are combined, washed with water, brine and dried over $MgSO_4$ and the solvent is removed under reduced pressure to give a brown oil. The crude product is purified by flash chromatography (gradient from iso-hexane to 1:1 isohexane: EtOAc), to afford 4-(3-methanesulfonyl -phenyl)-1*H*-pyrrole-3-carbonitrile; $[M-H]^-$ 245.

e) To an ice-cooled stirring suspension of NaH (60% dispersion in mineral oil; 48.8 mg, 1.22 mmol) in DMF (3 mL) is added dropwise and under inert atmosphere a solution of 4-(3-methanesulfonyl -phenyl)-1*H*-pyrrole-3-carbonitrile (0.2 g, 0.814 mmol) in DMF (2mL). After 10 minutes at 0˚C and 50 minutes at room temperature, methyl-2-bromoacetate (92 μL, 0.976 mmol) is added at 0˚C and the reaction mixture is left to stir at room temperature overnight. The mixture is partitioned between water and EtOAc. The organic layer is washed with water and brine then dried over $MgSO_4$. The solvent is removed under reduced pressure to give a yellow oil which is purified by flash chromatography (gradient from iso-hexane to 7:3 isohexane:EtOAc), to afford [3-cyano-4-(3-methanesulfonyl-phenyl)-pyrrol-1-yl]-acetic acid methyl ester, $[M-H]^-$ 317.

f) 1 M Aqueous LiOH (189 μL, 0.189 mmol) is added dropwise at 0˚C to a solution of [3-cyano-4-(3-methanesulfonyl-phenyl)-pyrrol-1-yl]-acetic acid methyl ester (60 mg, 0.189 mmol) in 1:1 THF:$H_2O$ (4 mL). The reaction mixture is stirred at 0˚C for 30 minutes then at room temperature for 5 hours. DCM is added and the aqueous layer is isolated, acidified to pH 1 with 1 M HCl and extracted with DCM. The organic phase is evaporated to afford [3-cyano-4-(3-methanesuffonyl-phenyl)-pyrrol-1-yl]-acetic acid as a white solid;

[M-H]- 303.

**Example 29 Preparation of [3-(3-chloro-5-cyano-phenyl)-4-cyano-pyrrol-1-yl]-acetic acid**

**a) 3-Chloro-5-(2-cyano-vinyl)-benzonitrile:**

A reaction mixture comprising commercially-available 3-chloro-5-iodobenzonitrile (1 g, 3.79 mmol), tetrabutylammonium iodide (1.39 g, 3.79 mmol), palladium (II) acetate (42.6 mg, 0.19 mmol), potassium carbonate (1.57 g, 11.3 mmol) in DMF (10 mL) is treated with acrylonitrile (0.375 mL, 5.69 mmol) and heated using microwave radiation in a Personal Chemistry Emrys™ Optimizer microwave reactor at 100˚C for 5 hours. The mixture is poured into water (100 mL) and extracted with EtOAc (3 x 50 mL). The combined organic layers are washed with water (50 mL), brine (50 mL), dried over $MgSO_4$ and concentrated *in vacuo.* Purification of the resulting crude product by chromatography on silica eluting with iso-hexane/EtOAc (EtOAc increasing from 0-20%) yields the titled compound; $[M-H]^-$

**b) [3-(3-chloro-5-cyano-phenyl)-4-cyano-pyrrol-1-yl]-acetic acid**

The titled compound is prepared analogously to Example 1 by replacing 3,5-*bis*(trifluoromethyl)cinnamonitrile with 3-chloro-5-(2-cyano-vinyl)-benzonitrile; $(MH+CH_3CN)^+$ 327.

**Reference Example 30 Preparation of 4-(3,5-*bis*-trifluoromethyl-phenyl)-1-carboxymethyl-1*H* pyrrole-3-carboxylic acid methyl ester**

The titled compound is prepared analogously to Example 1 by replacing 3,5-*bis*(trifluoromethyl)cinnamonitrile with 3-(3,5-*bis*-trifluoromethyl-phenyl)-acrylic acid methyl ester (prepared according to Bioorg Med Chem, Vol. 12, No. 9, pp. 2021-2034 (2004).

**Reference Example 31 Preparation of 4-(3,5-*bis*-trifluoromethyl-phenyl)-1-carboxymethyl-1*H*-pyrrole-3-carboxylic acid**

To a solution of 4-(3,5-*bis*-trifluoromethyl-phenyl)-1-carboxymethyl-1*H*-pyrrole-3-carboxylic acid methyl ester (Example 30) (0.08 g, 0.02 mmol) in MeOH (2 mL) is added 1 M NaOH(1 mL) and the reaction mixture is heated using microwave radiation in a Personal Chemistry Emrys™ Optimizer microwave reactor at 60˚C for 30 minutes. After further stirring at RT for 2 days, the pH of the mixture is adjusted to pH 5-6 using 1 M HCl and the product is extracted with DCM (3 x 3 mL). The organic portions are combined, washed with water (10 mL), brine (10 mL), dried ($MgSO_4$) and concentrated *in vacuo.* The resulting crude product is dissolved in EtOAc and triturated with iso-hexane to afford the titled compound as a white solid; $[M-H]^-$ 380.

**Reference Example 32 Preparation of [3-(3,5-*bis*-trifluoromethyl-phenyl)-4-dimethylcarbanioyl-pyrrol-1-yl]-acetic acid**

a) 3-(3,5-*bis*-trmuoromethyl-phenyl)-*N*,*N*-dimethyl-acrylamide:

To a solution of commercially-available (*E*)-3-(3,5-*bis*trifluoromethyl)cinammic acid (1.04 g, 3.65 mmol) in MeCN (10 mL) is added HOBt (0.56 g, 3.65 mmol) followed by PS-CDI (5.85 g of a 1.25 mmol/g resin, 7.31 mmol) and 2 M dimethylamine in THF (1.82 mL, 3.65 mmol). The reaction mixture is heated using microwave radiation in a Personal Chemistry Emrys™ Optimizer microwave reactor at 100˚C for 5 minutes and then filtered and washed through with DCM (5 mL), MeOH (5 mL), DCM (5 mL), MeOH (5 mL) again. The filtrate is concentrated *in vacuo* and the resulting crude product is dissolved in DCM (20 mL) and washed with saturated sodium bicarbonate solution (20 mL), water (20 mL), brine (20 mL), dried over $MgSO_4$ and concentrated *in vacuo* to afford 3-(3,5-*bis*-trifluoromethyl-phenyl)-*N,N*-dimethyl-acrylamide; $[M-H]^-$

b) [3-(3,5-*bis*-trifluoromethyl-phenyl)-4-dimethylcarbamoyl-pyrrol-1-yl]-acetic acid

The titled compound is prepared analogously to Example 1 by replacing 3,5-*bis*(trifluoromethyl)cinnamonitrile with 3-(3,5-*bis*-trifluoromethyl-phenyl)-*N*,*N*-dimethylacrylamide.

**Examples 33 and 36**

These examples namely,
[3-(3-chloro-5-trifluoromethyl-phenyl)-4-cyano-pyrrol-1-yl]-acetic acid (Example 33),
(3-cyano-4-pyridin-3-yl-pyrrol-1-yl)-acetic acid (Example 34),
3-cyano-4-(3,5-dimethoxy-phenyl)-pyrrol-1-yl]-acetic acid (Example 35) and
(3-benzofuran-2-yl-4-cyano-pyrrol-1-yl)-acetic acid (Example 36), are prepared by similar processes as that described in Example 2.

**Example 37 Preparation of [3-cyano-4-(3-iodo-5,trifluoromethyl-phenyl)-pyrrol-1-yl-acetic acid**

**a) (3-iodo-5-trifluoromethyl-phenyl)-methanol**

A cooled (-78˚C) solution of 3-iodo-5-trifluoromethyl-benzoic acid methyl ester (EP 612723) (1 g, 3.03 mmol) in toluene (20 mL) under an inert atmosphere of Argon is treated dropwise with 1.5 M DIBAL-H (4.44 mL, 6.67 mmol) over 5 minutes. The presuming pale yellow mixture is stirred at -78˚C for 1 hour and then at RT overnight. 1 M HCl (7 mL) is added to the reaction mixture which results in the formation of a white precipitate. The mixture is diluted with water (50 mL) and extracted with EtOAc (3 x 50 mL). The organic portions are combined, washed with water (50 mL), brine (50 mL), dried over $MgSO_4$ and concentrated *in vacuo.* The resulting off white crystalline solid is dried under vacuum at RT to yield the titled compound which is used in the next step without further purification.

**b) 3-iodo-5-trifluoromethyl-benzaldehyde**

To a cooled (-78˚C) solution of oxalyl chloride (0.612 mL, 7.02 mmol) in DMSO (1.1 mL, 15.6 mmol) and DCM (5 mL) which has pre-stirred for 40 minutes under an inert atmosphere of Argon is added dropwise a cooled (-78˚C) solution of (3-iodo-5-trifluoromethyl-phenyl)-methanol (0.943 g, 3.12 mL) in DCM (5 mL). After stirring at approximately -70˚C for 8 hours, the reaction mixture is treated with $Et_3N$ (2.23 mL, 15.9 mmol) and then allowed to warm to RT while stirring overnight. The mixture is partitioned between water and EtOAc. The organic layer is washed with water (50 mL) and brine (50 mL) then dried over $MgSO_4$. The solvent is removed *in vacuo* to afford the titled compound as an oil which crystallizes on standing.

c) [3-cyano-4-(3-iodo-5-trifluoromethyl-phenyl)-pyrrol-1-yl]-acetic acid

The titled compound is prepared analogously to Example 2 by replacing 3,5-dichlorobenzaldehyde with 3-iodo-5-trifluoromethyl-benzaldehyde; $[M-H]^-$ 419.

**Example 38 Preparation of [3-cyano-4-(3-cyano-5-trifluoromethyl-phenyl)-pyrrol-1-yl]-acetic acid**

**a) 3-cyano-4-(3-iodo-5-trifluoromethyl-phenyl)-pyrrol-1-yl]-acetic acid methyl ester**

The titled compound is prepared analogously to [3-cyano-4-(3,5-dichloro-phenyl)-pyrrol-1-yl]-acetic acid methyl ester (an intermediate in Example 2) by replacing 3,5-dichlorobenzaldehyde with 3-iodo-5-trifluoromethyl-benzaldehyde (Intermediate 37b).

**b) (3-cyano-4-(3-cyano-5-trifluoromethyl-phenyl)-pyrrol-1-yl]-acetic acid methyl ester**

A solution of 3-cyano-4-(3-iodo-5-trifluoromethyl-phenyl)-pyrrol-1-yl]-acetic acid methyl ester (0.2 g, 0.46 mmol) in DMF (5 mL) is treated with copper cyanide (0.046 g, 0.51 mmol) followed by a single crystal of sodium cyanide (approximately 4 mg). After stirring under an inert atmosphere of Argon at 105˚C for 2 days, the reaction mixture is left to cool to RT. Purification is carried out using mass directed preparative LC-MS eluting with acetonitrile:water to afford the titled compound; $[M-H]^-$ 332.

**c) [3-cyano-4-(3-cyano-5-trifluoromethyl-phenyl)-pyrrol-1-yl]-acetic acid**

1 M Aqueous NaOH (66 μL, 0.066 mmol) is added dropwise to a cooled (0˚C) solution of [3-cyano-4-(3-cyano-5-trifluoromethyl-phenyl)-pyrrol-1-yl]-acetic acid methyl ester (22 mg, 0.066 mmol) in 1:1 THF:$H_2O$ (4 mL). After stirring at 0˚C for 5 minutes, the reaction mixture is stirred at RT for 20 minutes. DCM (4 mL) is added and the mixture stirred for a further 5 minutes. The aqueous layer is isolated, acidified to pH 3-4 with 1 M HCl and extracted with DCM (2 x 3 mL). The organic phase is concentrated *in vacuo* and the resulting solid is dissolved in EtOAc (1 mL) and triturated with iso-hexane to yield the titled compound as an off-white solid; $[M-H]^-$ 318.

**Example 39 Preparation of [3-cyano-4-(3-ethanesulfanyl-5-trifluoromethyl-phenyl)-pyrrol-1-yl]-acetic acid**

**a) [3-cyano-4-(3-nitro-5-trifluorornethyl-phenyl)-pyrrol-1-yl]-acetic acid methyl ester**

The titled compound is prepared analogously to [3-cyano-4-(3-methanesulfonylphenyl)-pyrrol-1-yl]-acetic acid methyl ester (Intermediate **28e**) by replacing 3-methanesulfonylbenzoic acid with 3-nitro-5-trifluoromethyl-benzoic acid.

**b) [3-(3-amino-5-trffluoromethyl-phenyl)4-cyano-pyrrol-1-yl-acetic acid methyl ester**

A suspension comprising [3-cyano-4-(3-nitro-5-trifluoromethyl-phenyl)-pyrrol-1-yl)-acetic acid methyl ester (0.897 g, 2.54 mmol) and iron powder (0.709 g, 12.7 mmol) in dry ethanol (3 mL) and AcOH (5 mL) under an inert atmosphere of Argon is heated at 135˚C for 1 hour. After cooling to RT, the solvent is removed *in vacuo* and the resulting crude residue is partitioned between EtOAc and saturated sodium bicarbonate solution. The aqueous phase is extracted with EtOAc and the organic phases are combined, washed with saturated sodium bicarbonate solution, brine and dried over $MgSO_4$ and the solvent is removed *in vacuo* to give a brown solid. The crude product is purified by flash chromatography on silica eluting with 9:1 iso-hexane:EtOAc to afford the titled product; $[M-H]^-$ 322.

**c) [3-cyano4-(3-iodo-5-trifluoromethyl-phenyl)-pyrrol-1-yl]-acetic acid methyl ester**

A cooled (0˚C) solution of [3-(3-amino-5-trifluoromethyl-phenyl)-4-cyano-pyrrol-1-yl]-acetic acid methyl ester (0.396 g, 1.22 mmol) in concentrated HCl (10 mL) is treated dropwise with sodium nitrite (0.089 g, 1.29 mmol) in water (0.5 mL). After stirring at 0˚C for 1 hour, the reaction mixture is treated with potassium iodide (0.244 g, 1.47 mmol) and then left stirring at RT overnight. The resulting solution is extracted with EtOAc (3 x 50 mL) and the combined organic portions are treated with 1 M NaOH such that the pH is adjusted to pH 12. The organic portions are washed with water, brine, dried over $MgSO_4$ and the solvent is removed *in vacuo* to give the titled product; $[M-H]^-$ 433**.**

**d) [3-cyano-4-(3-ethanesulfonyl-5-trifluoromethyl-phenyl)-pyrrol-1-yl]-acetic acid**

A solution of [3-cyano-4-(3-iodo-5-trifluoromethyl-phenyl)-pyrrol-1-yl]-acetic acid methyl ester (0.263 g, 0.606 mmol) in DMSO (5 mL) under an inert atmosphere of Argon is treated with sodium ethanesulfinate (prepared according to J Med Chem, Vol. 32, pp. 2436-2442 (1989) (0.282 g, 2.43 mmol) and copper (I) iodide (0.462 g, 2.43 mmol). The resulting suspension is heated at 120˚C overnight and after cooling, the reaction mixture is treated with 2 M NaOH (0.606 mL, 0.606 mmol). After stirring at RT for 2 days, 2 M NaOH (0.606 mL, 0.606 mmol) is added, followed by water. DCM is added and the aqueous layer is isolated, acidified to pH 1 with 1 M HCl and extracted with DCM. The organic phase is dried over $MgSO_4$ and concentrated *in vacuo.* Purification is carried out using mass directed preparative LC-MS eluting with MeCN:water to afford an solid which is re-crystallized from 1:1 EtOH:water to afford the titled compound; $[M-H]^-$ 385.

**<u>Example 40</u> Preparation of (3-cyano-4-[3-(piperidine-1-sulfonyl)-5-trifluoromethyl-phenyl]-pyrrol-1-yl)-acetic acid**

**a) [3-(3-chlorosulfonyl-5-trifluoromethyl-phenyl)-4-cyano-pyrrol-1-yl]-acetic acid methyl ester**

A cooled (0˚C) solution of [3-(3-amino-5-trifluoromethyl-phenyl)-4-cyano-pyrrol-1-yl]-acetic acid methyl ester (Intermediate **<u>39b</u>**) (0.527 g, 1.49 mmol) in AcOH (15 mL) and concentrated HCl (5 mlL is treated dropwise with a solution of sodium nitrite (0.103 g, 1.49 mmol) in water (2 mL). After stirring at 0˚C for 1 hour, the reaction mixture is added to a stirred solution of $S0_2$/AcOH/$CuCl_2$/$H_2O$ (50 mL) (the preparation of the reagent is described below). The reaction mixture is allowed to warm to RT and is stirred overnight. The reaction mixture is then poured into water (200 mL) and extracted with EtOAc (3 x 75 mL). The combined organic layers are washed with water (2 x 70 mL) followed by brine (200 mL) and dried over $MgSO_4$. After filtration the solvent is removed *in vacuo* to afford the titled compound.

Preparation of the reagent $SO_2$/AcOH/$CuCl_{2/}H_2O$:

According to the reported procedure (E. E. Gilbert, Synthesis, 1-10, p6 (1969), glacial AcOH (100 mL) vigorously stirred at RT is treated by bubbling $SO_2$ gas. Once a saturated solution is achieved (approximately 10 g per 100 mL), the solution is treated with $CuCl_2$ (4 g) in water (5 mL). The resulting mixture is allowed to settle to give a green solution.

**b) (3-cyano-4-[3-(piperidine-4-sulfonyl)-5-trifluoromethyl-phenyl]-pyrrol-1-yl)-acetic acid**

A solution of [3-(3-chlorosulfonyl-5-triftuoromethyl-phenyl)-4-cyano-pyrrol-1-yl]-1-acetic acid methyl ester (0.166 g, 0.408 mmol) in THF (3 mL) is treated with piperidine (0.042 mL, 0.489 mmol) and $Et_3N$ (0.069 mL, 0.489 mmol). After stirring at RT for 4 hours, 1 M LiOH (0.408 mL, 0.408 mmol) is added and the reaction mixture is left stirring for 2 days. Water is added and then the reaction mixture is loaded on a phase separation column and washed with DCM. The mixture is acidified to pH 1 with 1 M HCl and extracted with DCM. The combined organic portions are concentrated *in vacuo* and purification using mass directed preparative LC-MS eluting with MeCN:water affords the titled compound; $[M-H]^-$ 440.

**<u>Examples 41 to 50</u>**

These examples namely,
{3-cyano-4-[3-(ethyl-methyl-sulfamoyl)-5-trifluoromethyl-phenyl]-pyrrol-1-yl}-acetic acid (Example 41),
{3-cyano-4-[3-(pyrrolidine-1-sulfony)l)-5-trifluoromethyl-phenyl]-pyrrol-1-yl}-acetic acid (Example 42),
[3-cyano-4-(3-diethylsulfamoyl-5-trifluoromethyl-phenyl)-pyrrol-1-yl]-acetic acid (Example 43),
(3-cyano-4-{3-[4-(2-cyano-ethyl)-piperazine-1-sulfonyl]-5-trifluoromethyl-phenyl}-pyrrol-1-yl)-acetic acid (Example 44),
[3-(3-butylsulfamoyl-5-trifluoromethyl-phenyl)-4-cyano-pyrrol-1-yl]-acetic acid (Example 45),
3-cyano-4-{3-[(2-cyano-ethyl)-methyl-sulfamoyl]-5-trifluoromethyl-phenyl}-pyrrol-1-yl)-acetic acid (Example 46),

{3-cyano-4-[3-(morpholine-4-sulfonyl)-5-trifluoromethyl-phenyl]-pyrrol-1-yl}-acetic acid (Example 47).
{3-cyano-4-[3-(2,2.dimethyl-propylsulfamoyl)-5-trifluoromethyl-phenyl]-pyrrol-1-yl}-acetic acid (Example 48).
{3-[3-(butyl-methyl-sulfamoyl)-5-trifluoromethyl-phenyl]-4-cyano-pyrrol-1-yl}-acetic acid (Example 49),
[3-cyano-4-(3-cyclobutylsulfamoyl-5-trifluoromethyl-phenyt)-pyrrol-1-yl]-acetic acid (Examples 50) and
[3-cyano-4-(3-cyclohexylsulfamoyt-5-trifluoromethyl-phenyl)-pyrrol-1-yl]-acetic acid (Example 51),
are prepared by similar processes as that described in Example 40.

**Example 52 Preparation of 3-cyano-4-(3-trifluoromethanesulfonyl-phenyl)-pyrrol-1-yl]-acetic acid**

**a) [3-cyano-4-(3-trifluoromethylsuffanyl-phenyl)-pyrrol-1-yl]-acetic acid methyl ester**

The titled compound is prepared analogously to [3-cyano-4-(3,5-dichloro-phenyl)-pyrrol-1-yl]-acetic acid methyl ester (Example 2c) by replacing 3,5-dichlorobenzaldehyde with 3-trifluoromethylsulfanyl-benzaldehyde.

**b) [3-cyano-4-(3-trifluoromethanesulfonyl-phenyl)-pyrrol-1-yl]-acetic acid methyl ester**

To a solution of [3-cyano-4-(3-trifluoromethylsutfanyl-phenyl)-pyrrol-1-yl]-acetic acid methyl ester (0.221 g, 0.65 mmol) in MeCN (2 mL) is added water (2 mL) followed by sodium periodate (0.417 g, 1.95 mmol) and ruthenium (III) chloride hydrate (6 mg, 0.0325 mmol). The reaction mixture is stirred at 10-15˚C for 30 minutes and then at RT for 4 hours. The mixture is partitioned between water and EtOAc. The organic layer is washed with brine, dried over $MgSO_4$ and concentrated *in vacuo.* The resulting crude product is purified by chromatography on silica eluting with *iso*-hexane/ EtOAc (EtOAc increasing from 0-20%) to yield the titled compound; $[M-H]^-$ 371.

**c) 3-cyano-4-(3-trifluoromethanesulfonyl-phenyl)-pyrrol-1-yl]-acetic acid**

1 M Aqueous NaOH (0.2 mL, 0.193 mmol) is added dropwise to a cooled (0˚C) solution of [3-cyano-4-(3-trifluoromethanesulfonyl-phenyl)-pyrrol-1-yl]-acetic acid methyl ester (72 mg, 0.193 mmol) in 1:1 THF:$H_2O$ (4 mL). After stirring at RT for 10 minutes, DCM (4 mL) is added and the mixture stirred for 5 minutes. The aqueous layer is isolated, acidified to pH 3-4 with 1 M HCl and extracted with DCM. The organic phase is concentrated *in vacuo* and purification of the residue by mass directed preparative LC-MS eluting with MeCN:water affords the titled compound; $[M-H]^-$ 357.

**Examples 53 Preparation of {3-[3-chloro-5-(piperldine-1-sulfonyl)-phenyl]-4-cyano-pyrrol-1-yl}-acetic acid**

**a) [3-(3-chloro-5-nitro-phenyl)-4-cyano-pyrrol-1-yl]-acetic acid methyl ester**

The titled compound is prepared analogously to [3-cyano-4-(3-methanesulfonylphenyl)-pyrrol-1-yl]-acetic acid methyl ester (intermediate **28e**) by replacing 3-methanesulfonylbenzoic acid with 3-chloro-5-nitro-benzaldehyde (WO 2005/037196).

**b) [3-(3-amino-5-chloro-phenyl)-4-cyano-pyrrol-1-yl]-acetic acid methyl ester**

A suspension of [3-(3-chloro-5-nitro-phenyl)-4-cyano-pyrrol-1-yl]-acetic acid methyl ester (0.16 g, 0.5 mmol) in ethanol (10 mL) is treated with tin (II) chloride (0.56 g, 2.5 mmol) and then stirred at 70˚C for 1 hour. The reaction mixture is allowed to cool to RT and poured onto ice (ca. 20 g). The pH of the solution is adjusted to pH 7-8 by addition of 5% sodium hydrogen carbonate and the resulting emulsion is filtered and extracted with EtOAc (3 x 50 mL). The combined organic portions are washed with brine (50 mL), dried over $MgSO_4$ and concentrated *in vacuo* to yield the titled compound as a pale red, oily solid; $[M-H]^-$ 288.

**c) {3-(3-chloro-5-(piperidine-1-sulfonyl)-phenyl]-4-cyano-pyrrol-1-yl}-acetic acid**

The titled compound is prepared analogously to {3-cyano-4-[3-(piperidine-1-sulfonyl)-5-trifluoromethyl-phenyl]-pyrrol-1-yl}-acetic acid (Example 40) by replacing [3-(3-amino-5-trifluoromethyl-phenyl)-4-cyano-pyrrol-1-yl]-acetic acid methyl ester (Intermediate 39b) with [3-(3-amino-5-chloro-phenyl)-4-cyano-pyrrol-1-yl]-acetic acid methyl ester

**Examples 54 to 59**

These examples namely,
{3-(3-chloro-5-(pyrrolidine-1-sulfonyl)-phenyl]-4-cyano-pyrrol-1-yl}-acetic acid (Example 54),

3-(3-butylsulfamoyl-5-chloro-phenyl)-4-cyano-pyrrol-1-yl]-acetic acid (Example 55),
{3-[3-chloro-5-(morpholine-4-sulfonyl)-phenyl]-4-cyano-pyrrol-1-yl]-acetic acid (Example 56),
(3-{3-chloro-5-[4-(2-cyano-ethyl)-piperazine-1-sulfonyl]-phenyl}-4-cyano-pyrrol-1-yl)-acetic acid (Example 57),
[3-(3-chloro-5-ethanesulfonyl-phenyl)-4-cyano-pyrrol-1-yl]-acetic acid (Example 58), and
(3-{3-chloro-5-[(2-hydroxy-ethyl)-methyl-sulfamoyl]-phenyl}-4-cyano-pyrrol-1-yl)-acetic acid (Example 59),
are prepared by similar processes as that described in Example 53.

**Example 60 Preparation of (3-[3-(butane-1-sulfonyl)-5-trifluoromethyl-phenyl]-4-cyano-pyrrol-1-yl}-acetic acid**

**a) {3-[3-(butane-1-sulfonyl)-5-trifluoromethyl-phenyl]-4-cyano-pyrrol-1-yl}-acetic acid methyl ester**

The titled compound is prepared analogously to [3-cyano-4-(3-ethanesulfonyl-5-trifluoromethyl-phenyl)-pyrrol-1-yl]-acetic acid (Example 39) by replacing sodium ethanesutfinate (prepared according to J Med Chem, Vol. 32, pp. 2436-2442 (1989) with sodium butanesulfinate.

**b) {3-[3-(butane-1-sulfonyl)-5-trifluoromethyl-phenyl]-4-cyano-pyrrol-1-yl}-acetic acid**

1 M Aqueous LiOH (270 μL, 0.270 mmol) is added dropwise at 0˚C to a solution of (3-13-(butane-1-sulfonyl)-5-trffluoromethyl-phenyl]-4-cyano-pyrrol-1-yl}-acetic acid methyl ester (116 mg, 0.270 mmol) in 1:1 THF:$H_2O$ (4 mL). The reaction mixture is stirred at RT for 6 hours. DCM is added and the aqueous layer is isolated, acidified to pH 1 with 1 M HCl and extracted with DCM. The organic phase is evaporated to afford a yellow crude solid which is re-crystallized from 1:1 EtOH:water to yield the titled compound as a white crystalline solid;
$[M-H]^-$ 413.

**Example 61 Preparation of {3-cyano-4-13-(propane-1-sulfonyl)-5-trifluoromethylphenyl]-pyrrol-1-yl}-acetic acid**

The titled compound is prepared analogously to Example 60 by replacing sodium butanesulfinate (prepared according to J Med Chem, Vol. 32, pp. 2436-2442 (1989) with sodium propane sulfinate.

**Example 62 Preparation of [3-cyano-4-(4'-ftuoro-5-trifluoromethyl-biphenyl-3-yl)-pyrrol-1-yl]-acetic acid**

A degassed solution comprising [3-cyano-4-(3-iodo-5-trifluoromethyl-phenyl)-pyrrol-1-yl]-acetic acid methyl ester (Intermediate **39d**) (0.3 g, 0.691 mmol) in dioxane (2 mL) at RT is treated with 4-fluorophenylboronic acid (0.106 g, 0.760 mmol) followed by sodium carbonate (0.146 g, 1.38 mmol) and palladium tetrakis(triphenyl phosphine) (0.039 g, 0.035 mmol) and water (0.3 mL). The reaction mixture is heated using microwave radiation in a Personal Chemistry Emrys™ Optimizer microwave reactor at 120˚C for 2 hours and then water and 2 M NaOH (2 mL) is added. The aqueous is isolated, washed with DCM and then acidified to pH 1 using 1 M HCl. The aqueous is extracted with DCM and the organic portions are washed with water and brine then dried over $MgSO_4$. The solvent is removed under reduced pressure to give a brown oil which is purified by flash chromatography eluting with 2% MeOH/DCM increasing to 10% MeOH/DCM to afford a brown solid. Re-crystallization of the product from EtOAc yields the titled compound as a pale pink solid; $[M-H]^-$ 387.

**Examote 63 Preparation of (3-(3-chloro-5-dimethylsulfamoyl-phenyl)4-cyano-pyrrol-1-yl]-acetic acid**

**a) 3-chloro-5-chlorosulfonyl-benzoic acid methyl ester:**

A cooled (0˚C) solution of 3-amino-5-chloro-benzoic acid methyl ester (WO 2004/014382) (2.27 g, 12.2 mmol) in AcOH (50 mL) and concentrated HCl (25 mL) is treated dropwise with a solution of sodium nitrite (0.843 g, 12.2 mmol) in water (2 mL). After stirring at 0˚C for 1 hour, the reaction mixture is added to a stirred solution of $SO_2$/AcOH/$CuCl_2$/$H_2O$ (100 mL) (the preparation of the reagent is described herein). The reaction mixture is allowed to warm to RT and is stirred overnight. The reaction mixture is then poured into water (200 mL) and extracted with EtOAc (2 x 200 mL). The combined organic layers are washed with water (2 x 70 mL) followed by brine (200 mL) and dried over $MgSO_4$. After filtration the solvent is removed *in vacuo* to afford the titled compound which is used in the next step without purification.

**b) 3-chloro-5-dimethylsulfamoyl-benzoic acid methyl ester**

A suspension of 3-chloro-5-chlorosuffonyl-benzoic acid methyl ester (0.48 g, 1.78 mmol) in THF (10 mL) under

an inert atmosphere of Argon is treated with 2 M dimethylamine in THF (1.07 mL, 2.14 mmol) followed by $Et_3N$ (0.3 mL, 2.14 mmol). After stirring at RT for 1.5 hours, the solvent is removed *in vacuo* and the crude product is dissolved in EtOAc. This organic portion is washed with water (50 mL), brine (50 mL), dried over $MgSO_4$ and concentrated *in vacuo* to afford the titled compound as an oily solid which crystallizes upon standing; $[M+H]^+$ 278.

**c) (3-(3-chloro-5-dimethylsulfamoyl-phenyl)-4-cyano-pyrrol-1-yl]-acetic acid**

The titled compound is prepared analogously to [3-cyano-4-(3-iodo-5-trifluoromethylphenyl)-pyrrol-1-yl]-acetic acid (Example 37) by replacing 3-iodo-5-trifluoromethyl-benzoic acid methyl ester (EP 612723) with 3-chloro-5-dimethylsulfamoyl-benzoic acid methyl ester.

**Examples 6 Preparation of [3-cyano-4-(3-dimethylsulfamoyl-5-trifluoromethylphenyl)-pyrrol-1-yl]-acetic acid**

The titled compound is prepared analogously to (3-(3-chforo-5-dimethylsulfamoylphenyl)-4-cyano-pyrrol-1-yl]-acetic acid (Example 63) by replacing 3-amino-5-chloro-benzoic acid methyl ester (WO 2004/014382) with 3-amino-5-trifluoromethyl-benzoic acid methyl ester (WO 20041014382).

**Example 65 Preparation of (3-cyano-4-[3-(propane-2-sulfonyl)-5-trifluoromethylphenyl]-pyrrol-1-yl}-acetic acid**

**a) 3-chlorosulfonyl-6-trifluoromethyl-benzoic acid methyl ester**

The titled compound is prepared analogously to 3-chloro-5-chlorosulfonyl-benzoic acid methyl ester (Example 63a) by replacing 3-amino-5-chloro-benzoic acid methyl ester (WO 2004/014382) with 3-amino-5-trifluoromethyl-benzoic acid methyl ester (WO 2004/014382).

**b) 3-(Propane-2-sulfonyl)-5-trifluoromethyl-benzoic acid methyl ester**

A solution of 3-chlorosulfonyl-5-trifluoromethyl-benioic acid methyl ester (1.31 g, 4.34 mmol) in dioxane (30 mL) is treated with sodium sulphite (1.a9 g, 8.68 mmol) and sodium hydrogen carbonate (0.73 g, 8.68 mmol) and allowed to stir at 80˚C for 2 hours. After cooling to RT, the solvent is removed *in vacuo* and the crude residue is dissolved in DMF (15 mL) under an inert atmosphere of Argon. To the resulting yellow suspension is added dropwise 2-iodopropane (0.87 mL, 8.68 mmol) at RT. The reaction mixture is sonicated for 5 minutes and then stirred at RT overnight Water (70 mL) is added to the reaction mixture and the aqueous is extracted with EtOAc (3 x 50 mL). The organic portions are combined, washed with water, brine, dried over $MgSO_4$ and the solvent is removed under reduced pressure to give an orange solid. The crude product is purified by chromatography on silica (eluent gradient from iso-hexane to 1:1 iso-hexane:EtOAc) to afford the titled compound; $[M-H]^-$ 295/309.

**c) {3-cyano-4-[3-(propane-2-sulfonyl)-5-trifluoromethyl-phenyl]-pyrrol-1-yl}-acetic acid**

The titled compound is prepared analogously to (3-(3-chloro-5-dimethylsulfamoylphenyl)-4-cyano-pyrrol-1-yl]-acetic acid (Example 63) by replacing 3-chloro-5-dimethylsutfamoyl-benzoic acid methyl ester (Example 63b) with 3-(propane-2-sulfonyl)-5-trifluoromethyl-benzoic acid methyl ester (Example 65b).

**Example 66 Preparation of 13-cyano-4-(3-methanesulfonyl-5-trifluoromethyl-phenyl)pyrrol-1-yl]-acetic acid**

The titled compound is prepared analogously to (3-cyano-4-[3-(propane-2-sulfonyl)-5-trifluoromethyl-phenyl]-pyrrol-1-yl)-acetic acid (Example 65) by replacing 2-iodopropane with methyl iodide.

**Example 67 Preparation of 2-[3-(3,5-*bis*-trifluoromethyl-phenyl)-4-cyano-pyrrol-1-yl]propionic acid**

The titled compound is prepared analogously to [3-(3.5-*bis*-trifluoromethyl-phenyl)-4-cyano-pyrrol-1-yl]-acetic acid (Example 1) by replacing methyl-2-bromoacetate with 2-bromopropionic acid ethyl ester.

*Pharmaceutical Use and Assay*

Compounds of formula (I) and (Ia) and their pharmaceutically acceptable salts, hereinafter referred to alternatively as "agents of the invention", are useful as pharmaceuticals. In particular, the compounds have good CRTh2 receptor antagonist activity and may be tested in the following assays.

Filtration binding assay protocol

The binding of CRTh2 antagonists is determined using membranes prepared from human CRTh2-expressing Chinese Hamster Ovary cells (CHO.K1-CRTh2). To produce cell membranes CHO.K1-CRTh2 cells cultured in roller bottles are harvested using cell dissociation buffer (Invitrogen). The cells are pelleted by centrifugation (167 g, 5 min). The cell pellet is incubated in hypotonic buffer (15 mM Tris-OH, 2 mM $MgCl_2$, 0.3 mM EDTA, 1 mM EGTA, 1 x Complete™ tablet) at 4˚C for 30 minutes. At 4˚C cells are homogenized using a Polytron® (IKA Ultra Turrax T25) for 5 bursts of 1 second. The homogenate is centrifuged (Beckman Optima TM TL Ultracentrifuge, 48000 g, 30 minutes at 4˚C). The supernatant is discarded and the membrane pellet re-suspended in homogenisation buffer (75 mM Tris-OH, 12.5 mM $MgCl_2$, 0.3 mM EDTA, 1 mM EGTA, 250 mM Sucrose, 1 x Complete™ tablet Membrane preparations are aliquoted and stored at 80˚C. The protein content is estimated using Bradford Protein Assay Dye (Bio Rad).

The binding of $[^3H]$-$PGD_2$ (157 Ci/mmol) to CHO.K1-CRTh2 membranes is determined in the absence (total binding) and presence (non-specific binding) of unlabelled $PGD_2$ (1 µM). Subtraction of the cpm (counts per minute) of $[^3H]$-$PGD_2$ binding in presence of excess unlabelled $PGD_2$ from that observed in the absence of excess unlabelled $PGD_2$ is defined as specific binding. Active CRTh2 antagonists are able to compete with $[^3H]$-$PGD_2$ for binding to the CRTh2 receptor and are identified in a decrease in the number of cpm bound.

The assay is performed in Greiner U-bottomed 96 well-plates, in a final volume of 100 µL per well. CHO.K1-CRTh2 membranes were diluted in assay buffer (10 mM HEPES-KOH (pH 7.4), 1 mM EDTA and 10 mM $MnCl_2$) and 10 µg are added to each well $[^3H]$-$PGD_2$ is diluted in assay buffer and added to each well at a final concentration of 2.5 nM. To determine non-specific binding, PH]-$PGD_2$ binding to the CRTh2 receptor is competed with using unlabelled $PGD_2$ at a final well concentration of 1 µM. The experiment is done in triplicate, with reagents added to the wells as follows:

- 25 µL assay buffer for total binding or
- 25 µL $PGD_2$ to determine non-specific binding
- 25 µL $[^3$ H]$PGD_2$
- 50 µL membranes
- 25 µL test compound in DMSO/assay buffer

The plates are incubated at room temperature on a shaker for 1 hour, and then harvested (Tomtec Harvester 9600) onto GF/C filter plates using wash buffer (10 mM HEPES-KOH, pH 7.4). The plate is dried for 2 hours, prior to addition of Micro-Scint 20™ (50 µL) and sealing with TopSeal-S™. Plates are then counted using a Packard Top Count instrument, Plates are then read on the Packard Topcount with the 3H Scintillation program (1 min./well).

Ki (dissocation constant for the inhibition) values for the CRTh2 antagonists are reported. Ki values are determined using Sigma Plot™ software, using the Cheng-Prussoff equation.

$$Ki = IC_{50} / 1 + [S]/Kd$$

where S is the concentration of radioligand and Kd is the dissociation constant

CRTH2 cAMP functional assay protocol

This assay is conducted in CHO.K1-CRTh2 cells. cAMP is generated in the cell by stimulating cells with 5 µM forskolin, an adenylate cyclase activator. $PGD_2$ is added to activate the CRTh2 receptor which results in the attenuation of the forskolin-induced cAMP accumulation. Potential CRTh2 antagonists are tested for their ability to inhibit the $PGD_2$-mediated attenuation of the forskolin-induced cAMP accumulation in CHO.K1-CRTh2 cells.

For each concentration value on the dose-response curve, test compounds are prepared in assay stimulation buffer (HBSS, 5 mM HEPES, 10 µM IBMX ± 0.1% human serum albumin) containing DMSO (3% vol/vol) and 5 µL/well is added to an assay plate (384 well white optiplate).

CHO.K1-CRTh2 cultured in tissue culture flasks are washed with PBS and harvested with dissociation buffer. Cells are washed with PBS and re-suspended in stimulation buffer to a concentration of 0.4 x $10^6$/mL and added to the assay plate (10 µL/well).

The assay plate is incubated at room temperature on a shaker for 15 minutes.

A mix of agonist (10 nM Prostaglandin $D_2$) and 5 µM forskolin is prepared in assay stimulation buffer and added to the assay plate (5 µL/well).

In addition, a cAMP standard is serially diluted in assay stimulation buffer and added to separate empty wells on the assay plate (20 µl/well). The cAMP standard allows for the quantification of cAMP generated in CHO.K1-CRTH2

cells.

The assay plate is incubated at room temperature on a shaker for 60 minutes.

Cell lysis buffer (Lysis buffer: Milli-Q $H_2O$, 5 mM HEPES, 0.3% Tween-20, 0.1% human serum albumin) is added to a bead mix (containing Alphascreen™ anti-cAMP acceptor beads 0.06 units/μL, Alphascreen™ streptavidin-coated donor beads 0.06 units/μL, biotinylated cAMP 0.06 units/μL, 10 μM IBMX) is prepared under darkened conditions 60 minutes prior to addition to the assay plate. The resulting lysis mix is added to all wells of the assay plate (40 μL/well).

The assay plate is sealed with Topseal-S™ and incubated in the dark at room temperature on a shaker for 45 minutes. The plate is then counted using a Packard Fusion™ instrument.

The resulting counts per minute are converted to nM cAMP by using the prepared cAMP standard curve. $IC_{50}$ values (concentration of CRTh2 antagonist required to inhibit 50% of the $PGD_2$-mediated attenuation of forskolin-induced cAMP accumulation in CHO.K1-CRTh2 cells) are then determined using Prism™ software.

Compounds of the Examples, herein below, generally have Ki values in the filtration binding assay below 10 μM. For example, the compounds of Examples 1,2,3,8,12,15,17, 21, 25, 40, 42, 45, 50, 53 and 64 have Ki values of 0.010, 0.034, 0.244, 0.163, 0.172, 0.144, 0.089, 0.096, 0.040, 0.002, 0.006, 0.006, 0.004, 0.002 and 0.042 μM, respectively.

Compounds of the Examples, herein below, generally have $IC_{50}$ values in the functional assay below 10 μM. For example, the compounds of Examples 1, 2, 3, 8, 12, 15, 17, 21, 25, 40, 42, 45, 50, 53 and 64 have $IC_{50}$ values of 0.028, 0.057, 0.846, 0.255, 0.069, 0.282, 0.161, 0.147, 0.025, 0.012, 0.017, 0.016, 0.009, 0.046 and 0.014 μM, respectively.

Compounds of formula (I) and (Ia), in free or salt form, are antagonists of the G-protein-coupled receptor CRTh2, expressed on Th2 cells, eosinophils and basophils. $PGD_2$ is the natural ligand for CRTh2. Thus, antagonists which inhibit the binding of CRTh2 and $PGD_2$ are useful in the treatment of allergic and anti-inflammatory conditions. Treatment in accordance with the invention may be symptomatic or prophylactic.

Accordingly, agents of the invention are useful in the treatment of inflammatory or obstructive airways diseases, resulting, e.g., in reduction of tissue damage, airways inflammation, bronchial hyperreactivity, remodeling or disease progression. Inflammatory or obstructive airways diseases to which the present invention is applicable include asthma of whatever type or genesis including both intrinsic (non-allergic) asthma and extrinsic (allergic) asthma, mild asthma, moderate asthma, severe asthma, bronchitis asthma, exercise-induced asthma, occupational asthma and asthma induced following bacterial infection. Treatment of asthma is also to be understood as embracing treatment of subjects, e.g., of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g., of acute asthmatic or bronchoconstrictor attack, improvement in lung function or improved airways hyperreactivity. It may further be evidenced by reduced requirement for other, symptomatic therapy, i.e., therapy for or intended to restrict or abort symptomatic attack when it occurs, e.g., anti-inflammatory (e.g., corticosteroid) or bronchodilatory. Prophylactic benefit in asthma may, in particular, be apparent in subjects prone to "morning dipping". "Morning dipping" is a recognized asthmatic syndrome, common to a substantial percentage of asthmatics and characterized by asthma attack, e.g., between the hours of about 4-6 AM, i.e., at a time normally substantially distant from any previously administered symptomatic asthma therapy.

Other inflammatory or obstructive airways diseases and conditions to which the present invention is applicable include acute lung injury (ALI), adult respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular, other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis including, e.g., aluminosis, anthracosis, asbestosis, chalicosis, ptilosis, siderosis, silicosis, tabacosis and byssinosis.

Having regard to their anti-inflammatory activity, in particular, in relation to inhibition of eosinophil activation, agents of the invention are also useful in the treatment of eosinophil related disorders, e.g., eosinophilia, in particular, eosinophils-related disorders of the airways, e.g., involving morbid eosinophilic infiltration of pulmonary tissues including hypereosinophilia as it effects the airways and/or lungs, as well as, e.g., eosinophil-related disorders of the airways consequential or concomitant to Löffler's syndrome; eosinophilic pneumonia; parasitic, in particular, metazoan, infestation including tropical eosinophilia; bronchopulmonary aspergillosis; polyarteritis nodosa including Churg-Strauss syndrome; eosinophilic granuloma; and eosinophil-related disorders affecting the airways occasioned by drug-reaction.

Agents of the invention are also useful in the treatment of inflammatory or allergic conditions of the skin, e.g.,

psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphisus, epidermolysis bullosa acquisita and other inflammatory or allergic conditions of the skin.

Agents of the invention may also be used for the treatment of other diseases or conditions, in particular, diseases or conditions having an inflammatory component, e.g., treatment of diseases and conditions of the eye, such as conjunctivitis, keratoconjunctivitis sicca and vernal conjunctivitis; diseases affecting the nose including allergic rhinitis; and inflammatory disease, in which autoimmune reactions are implicated or having an autoimmune component or aetiology, including autoimmune hematological disorders, e.g., hemolytic anemia, aplastic anaemia, pure red cell anaemia and idiopathic thrombocytopenia; systemic lupus erythematosus; polychondritis; sclerodoma; Wegener granulamatosis; dermatomyositis; chronic active hepatitis; myasthenia gravis; Steven-Johnson syndrome; idiopathic sprue; autoimmune inflammatory bowel disease, e.g., ulcerative colitis and Crohn's disease; endocrine opthalmopathy; Grave's disease; sarcoidosis; alveolitis; chronic hypersensitivity pneumonitis; multiple sclerosis; primary billiary cirrhosis; uveitis (anterior and posterior); keratoconjunctivitis sicca and vernal keratoconjunctivitis; interstitial lung fibrosis; psoriatic arthritis; and glomerulonephritis, with and without nephrotic syndrome, e.g., including idiopathic nephrotic syndrome or minal change nephropathy.

Other diseases or conditions which may be treated with agents of the invention include septic shock; rheumatoid arthritis; osteoarthritis; proliferative diseases, such as cancer; atherosclerosis; allograft rejection following transplantation; stroke; obesity; restenosis; diabetes, e.g., diabetes mellitus type I (juvenile diabetes) and diabetes mellitus type II; diarrheal diseases; ischemia/reperfusion injuries; retinopathy, such as diabetic retinopathy or hyperbaric oxygen-induced retinopathy; and conditions characterized by elevated intraocular pressure or secretion of ocular aqueous humor, such as glaucoma.

The effectiveness of an agent of the invention in inhibiting inflammatory conditions, e.g., in inflammatory airways diseases, may be demonstrated in an animal model, e.g., a mouse or rat model, of airways inflammation or other inflammatory conditions, e.g., as described by Szarka et al., J Immunol Methods, Vol. 202, pp. 49-57 (1997); Renzi et al., Am Rev Respir Dis, Vol. 148, pp. 932-939 (1993); Tsuyuki et al., J Clin Invest, Vol. 96, pp. 2924-2931 (1995); Cemadas et al., Am J Respir Cell Mol Biol, Vol. 20, pp. 1-8 (1999); and Williams and Galli, J Exp Med, Vol. 192, pp. 455-462 (2000).

The agents of the invention are also useful as co-therapeutic agents for use in combination with other drug substances, such as anti-inflammatory, bronchodilatory or antihistamine drug substances, particularly in the treatment of obstructive or inflammatory airways diseases, such as those mentioned hereinbefore, e.g., as potentiators of therapeutic activity of such drugs or as a means of reducing required dosaging or potential side effects of such drugs. An agent of the invention may be mixed with the other drug substance in a fixed pharmaceutical composition or it may be administered separately, before, simultaneously with or after the other drug substance. Accordingly the invention includes a combination of an agent of the invention as hereinbefore described with an anti-inflammatory, bronchodilatory, antihistamine or anti-tussive drug substance, said agent of the invention and said drug substance being in the same or different pharmaceutical composition.

Such anti-inflammatory drugs include steroids, in particular, glucocorticosteroids, such as budesonide, beclamethasone dipropionate, fluticasone propionate, ciclesonide or mometasone furoate; or steroids, described in WO 02/88167, WO 02/12266, WO 02/100879, WO 02/00679 (especially those of Examples 3,11,14,17, 19, 26, 34,37,39,51,60,67, 72, 73, 90, 99 and 101), WO 03/035668, WO 03/048181,WO 03/062259, WO 03/064445 and WO 03/072592, WO 04/039827, WO 04/066920; non-steroidal glucocorticoid receptor agonists, such as those described in WO 00/00531, WO 02/10143, DE 10261874, WO 03/082280, WO 03/082787, WO 03/104195, WO 03/101932, WO 04/019935, WO 04/018429 WO 04/063163, WO 04/005229, WO 03/086294 and WO 04/26248, WO 04/071389; LTB4 antagonists, such as those described in U.S. Patent No. 5,451,700; LTD4 antagonists, such as montelukast and zafirlukast; PDE4 inhibitors, such as cilomilast (Ariflo® GlaxoSmithKline), Roflumilast (Byk Gulden),V-11294A (Napp), BAY19-8004 (Bayer), SCH-351591 (Schering-Plough), Arofylline (Almirall Prodesfarma), PD189659 (Parke-Davis), AWD-12-281 (Asta Medica), CDC-801 (Celgene), SelCID(TM) CC-10004 (Celgene), KW-4490 (Kyowa Hakko Kogyo), WO 03/104204, WO 03/104205, WO 04/000814, WO 04/000839 and WO 04/005258 (Merck), as well as those described in WO 98/18796 and WO 03/39544; A2a agonists, such as those described in EP 1052264, EP 1241176, EP 409595A2, WO 94/17090, WO 96/02543, WO 96/02553, WO 98/28319, WO 99/24449, WO 99/24450, WO 99/24451, WO 99/36877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462 and WO 03/086408; A2b antagonists, such as those described in WO 02/42298; and beta (β)-2-adrenoceptor agonists, such as albuterol (salbutamol), metaproterenol, terbutaline, salmeterol, fenoterol, procaterol, and especially, formoterol and pharmaceutically acceptable salts thereof, and compounds (in free or salt or solvate form) of formula (I) of WO 00/75114, which document is incorporated herein by reference, preferably compounds of the Examples thereof, especially a compound of formula

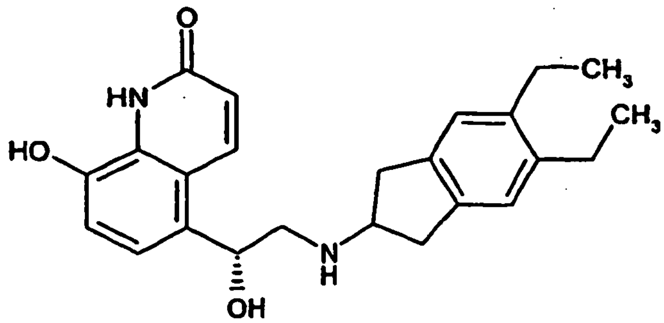

,

and pharmaceutically acceptable salts thereof, as well as compounds (in free or salt or solvate form) of formula (I) of WO 04/16601. Further β-2-adrenoreceptor agonists include compounds of JP 05025045, WO 93/18007, WO 99/64035, U.S. Patent No. 2002/0055651, WO 01/42193, WO 01/83462, WO 02/66422, WO 02/70490, WO 02/76933, WO 03/024439, WO 03/072539, WO 03/042160, WO 03/091204, WO 03/042164, WO 03/099764, WO 04/016578, WO 04/022547, WO 04/032921, WO 04/037773, WO 04/037807, WO 04/039762, WO 04/039766, WO 04/045618, WO 04/046083, WO 04/033412, WO 04/037768, WO 04/037773 and EP 1440966.

Such bronchodilatory drugs include anticholinergic or antimuscarinic agents, in particular, ipratropium bromide, oxitropium bromide, tiotropium salts and CHF 4226 (Chiesi), but also those described in WO 04/096800, WO 01/04118, WO 02/51841, WO 02/53564, WO 03/00840, WO 03/87094, WO 04/05285, WO 02/00652, WO 03/53966, EP 0424021, U.S. Patent No. 5,171,744, U.S. Patent No. 3,714,357 and WO 03/33495.

Such co-therapeutic antihistamine drug substances include cetirizine hydrochloride, acetaminophen, clemastine fumarate, promethazine, loratidine, desloratidine, diphenhydramine and fexofenadine hydrochloride.

Combinations of agents of the invention and steroids, β-2 agonists, PDE4 inhibitors or LTD4 antagonists may be used, e.g., in the treatment of COPD or, particularly, asthma. Combinations of agents of the invention and anticholinergic or antimuscarinic agents, PDE4 inhibitors, dopamine receptor agonists or LTB4 antagonists may be used, e.g., in the treatment of asthma or, particularly, COPD.

Other useful combinations of agents of the invention with anti-inflammatory drugs are those with antagonists of chemokine receptors, e.g., CCR-1, CCR-2, CCR-3, CCR-4, CCR-5, CCR-6, CCR-7, CCR-8, CCR-9, CCR-10, CXCR1, CXCR2, CXCR3, CXCR4 and CXCRS; particularly useful are CCR-3 antagonists, such as those described in WO 02/026723, especially 4-{3-[(S)-4-(3,4-dichlorobenzyl)-morpholin-2-ylmethyl]-ureidomethyl}-benzamide and those described in WO 03/077907, WO 03/007939 and WO 02/102775.

Also especially useful are CCR-5 antagonists, such as Schering-Plough antagonists SC-351125, SCH-55700 and SCH-D; Takeda antagonists, such as *N*-[[4-[[[6,7-dihydro-2-(4-methylphenyl)-5*H*-benzo-cyclohepten-8-yl]carbonyl] amino]phenyl]-methyl]tetrahydro-*N*,*N*-dimethyl-2H-pyran-4-aminium chloride (TAK-770); and CCR-5 antagonists, described in U.S. Patent No. 6,166,037, WO 00/66558 and WO 00/66559.

The agents of the invention may be administered by any appropriate route, e.g., orally, e.g., in the form of a tablet or capsule; parenterally, e.g., intravenously; by inhalation, e.g., in the treatment of inflammatory or obstructive airways disease; intranasally, e.g., in the treatment of allergic rhinitis; topically to the skin, e.g., in the treatment of atopic dermatitis; or rectally, e.g., in the treatment of inflammatory bowel disease.

The present invention also provides a pharmaceutical composition comprising a compound of formula (I), in free form or in the form of a pharmaceutically acceptable salt, optionally together with a pharmaceutically acceptable diluent or carrier therefore. The composition may contain a co-therapeutic agent, such as an anti-inflammatory, bronchodilatory or antihistamine drug, as hereinbefore described. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g., patches. Compositions for inhalation may comprise aerosol or other atomizable formulations or dry powder formulations.

The present invention also provides for the use of a compound of the present invention in any of the aforementioned embodiments, in free or pharmaceutically acceptable salt form, for the manufacture of a medicament for the treatment of an inflammatory or allergic condition, particularly an inflammatory or obstructive airways disease.

The present invention also provides a method for treating or preventing inflammatory or allergic conditions comprising administering to a patient in need thereof a therapeutically effective amount of a compound of the present invention, in free or a pharmaceutically acceptable salt form.

When the composition comprises an aerosol formulation, it preferably contains, e.g., a hydro-fluoro-alkane

(HFA) propellant, such as HFA134a or HFA227 or a mixture of these, and may contain one or more co-solvents known in the art, such as ethanol (up to 20% by weight); and/or one or more surfactants, such as oleic acid or sorbitan trioleate; and/or one or more bulking agents, such as lactose. When the composition comprises a dry powder formulation, it preferably contains, e.g., the compound of formula (I) having a particle diameter up to 10 microns, optionally together with a diluent or carrier, such as lactose, of the desired particle size distribution and a compound that helps to protect against product performance deterioration due to moisture. When the composition comprises a nebulized formulation, it preferably contains, e.g., the compound of formula (I), either dissolved or suspended, in a vehicle containing water, a co-solvent, such as ethanol or propylene glycol and a stabilizer, which may be a surfactant.

The invention includes:

(a) an agent of the invention in inhalable form, e.g., in an aerosol or other atomizable composition or in inhalable particulate, e.g., micronized form;
(b) an inhalable medicament comprising an agent of the invention in inhalable form;
(c) a pharmaceutical product comprising such an agent of the invention in inhalable form in association with an inhalation device; and
(d) an inhalation device containing an agent of the invention in inhalable form.

Dosages of agents of the invention employed in practicing the present invention will of course vary depending, e.g., on the particular condition to be treated, the effect desired and the mode of administration. In general, suitable daily dosages for oral administration are of the order of 0.01-100 mg/kg.

## Claims

**1.** A compound of formula (I)

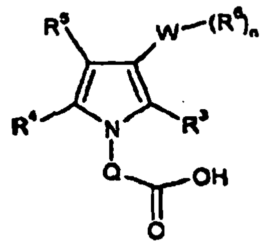

(I)

In free or pharmaceutically acceptable salt form,
wherein
Q is

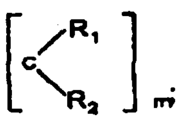

$R^1$ and $R^1$ are, independently, H, $C_1$-$C_6$-alkyl, or together with the carbon atom to which they are attached form a divalent $C_3$-$C_8$-cycloaliphatic group;
$R^3$ and $R^4$ are independently selected from H and $C_1$-$C_8$-alkyl;
$R^3$ is cyano;
$R^6$ is selected from H, halogen, $C_1$-$C_8$-alkyl, $C_1$-$C_8$-haloalkyl, a $C_3$-$C_{15}$-carbocyclic group, nitro, cyano, $SO_2R^{6a}$, $C_1$-$C_8$-alkylcarbonyl, $C_1$-$C_8$-alkoxycarbonyl, $C_1$-$C_8$-alkoxy, $C_1$-$C_8$-haloalkoxy, -$SR^{6b}$, carboxy, carboxy-$C_1$-$C_8$-alkyl, amino, amino($C_1$-$C_8$-alkyl), $C_1$-$C_8$-alkylamino($C_1$-$C_8$-alkyl), di($C_1$-$C_8$-alkyl)amino($C_1$-$C_8$-alkyl), $C_1$-$C_8$-alkylamino, di ($C_1$-$C_8$-alkyl)amino, $SO_2NR^{6c}R^{6d}$, -C(O)$NR^{6e}R^{6f}$, $C_1$-$C_8$-hydroxyalkyl, $NR^{6g}SO_2R^{6h}$, $NR^{6i}(CO)R^{6j}$, $SOR^{6k}$, a $C_8$-$C_{15}$ aromatic carbocyclic group and a 4- to 10-membered heterocyclic group having one or more heteroatoms selected from the group consisting of oxygen, nitrogen and sulphur;
$R^{6a}$, $R^{6k}$ and $R^{6b}$ are independently selected from $C_1$-$C_8$-alkyl, $C_1$-$C_8$-hydroxyalkyl, $C_1$-$C_8$-alkylamino($C_1$-$C_8$-alkyl), di($C_1$-$C_8$-alkyl)amino($C_1$-$C_8$-alkyl), $C_1$-$C_8$-cyanoalkyl, a $C_3$-$C_{15}$-carbocyclic group, $C_1$-$C_8$-haloalkyl and a 4- to 10-membered heterocyclic group having one or more heteroatoms selected from the group consisting of oxygen, nitrogen and sulphur;
$R^{6c}$, $R^{6d}$, $R^{6e}$ and $R^{6f}$ are independently H, $C_1$-$C_8$-alkyl, $C_1$-$C_8$-hydroxyalkyl, $C_1$-$C_8$-alkylamino($C_1$-$C_8$-alkyl), di($C_1$-$C_8$-alkyl)amino($C_1$-$C_8$-alkyl), $C_1$-$C_8$-cyanoalkyl, a $C_3$-$C_{15}$-carbocyclic group, $C_1$-$C_6$-haloalkyl, a 4- to 10-membered heterocyclic group having one or more heteroatoms selected from the group consisting of oxygen, nitrogen and sulphur, or together with the nitrogen atom to which they are attached form a $C_4$-$C_{10}$-heterocyclic group;
$R^{6g}$ and $R^{6l}$ are independently selected from H, $C_1$-$C_8$-alkyl, $C_1$-$C_8$-hydroxyalkyl, $C_1$-$C_8$-alkylamino($C_1$-$C_8$-alkyl), di($C_1$-$C_8$-alkyl)amino($C_1$-$C_8$-alkyl), $C_1$-$C_8$-cyanoalkyl, a $C_3$-$C_{15}$-carbocyclic group, $C_1$-$C_8$-haloalkyl and a 4- to 10-membered heterocyclic group having one or more heteroatoms selected from the group consisting of oxygen, nitrogen and sulphur;
$R^{6h}$ and $R^{6j}$ are independently selected from $C_1$-$C_8$-alkyl, a $C_3$-$C_{15}$-carbocyclic group, a 4-to 10-membered heterocyclic group having one or more heteroatoms selected from the group consisting of oxygen, nitrogen and sulphur, $C_1$-$C_8$-hydroxyalkyl, amino($C_1$-$C_8$ alkyl), $C_1$-$C_8$-alkylamino($C_1$-$C_8$-alkyl), di($C_1$-$C_8$-alkyl)amino($C_1$-$C_8$-alkyl), and $C_1$-$C_8$-cyanoalkyl;
W is selected from $C_3$-$C_{15}$-carbocyclic group and 4- to 10-membered heterocycle having one or more heteroatoms selected from the group consisting of oxygen, nitrogen and sulphur;
m is an integer selected from 1-3; and
n is an integer selected from 1-4;
with the proviso that the compounds
[3-cyano-4-(2,3-dichloro-phenyl)-pyrrol-1-yl]-acetic acid;
N-(2-carboxyethyl)-3-cyano-4-(2,3-dichloro-phenyl)pyrrole;
N-(3-carboxypropyl)-3-cyano-4-(2,3-dichloro-phenyl)pyrrole and N-(1-carboxyethyl)-3-cyano-4-(2,3-dichloro-phenyl)-pyrrole are excluded

**2.** A compound of formula (I) according to Claim 1, in free or pharmaceutically acceptable salt form, wherein the symbols have the following meanings independently, collectively or in any combination or sub-combination:

(i) $R^1$ and $R^2$ are, independently, H or $C_1$-$C_8$-alkyl;
(ii) $R^3$ and $R^4$ are H or $C_1$-$C_8$-alkyl:
(iii) $R^5$ is cyano;
(iv) $R^6$ is H, halogen $C_1$-$C_8$-alkyl, $C_1$-$C_8$-haloalkyl, cyano, $SO_2R^{6a}$ and $-SR^{6b}$;
(v) $R^{6a}$ and $R^{6b}$ are independently selected from $C_1$-$C_8$-alkyl, $C_3$-$C_{15}$-carbocyclic group, and $C_1$-$C_8$-haloalkyl;
(vi) W is a $C_3$-$C_{10}$-carbocyclic group or a 4- to 10-membered heterocyclic group having one or more ring oxygen atoms;
(vii) m is 1; and
(viii) n is an integer selected from 1-4.

**3.** A compound according to Claim 1, in free or pharmaceutically acceptable salt form, wherein the compound is of formula (Ia)

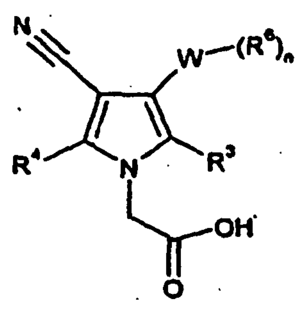

(Ia)

**4.** A compound according to Claim 3, in free or pharmaceutically acceptable salt form, wherein
$R^3$ and $R^4$ are H; and
$R^6$ is selected from H, halogen, $C_1$-$C_8$-haloalkyl,cyano, $SO_2R^{6a}$ and $-SR^{6b}$;
$R^{6a}$ and $R^{6b}$ are independently selected from $C_1$-$C_4$-alkyl, a $C_8$-$C_{10}$-carbocyclic group and $C_1$-$C_4$-haloalkyl; and
n is an integer selected from 2-3.

**5.** A compound according to Claim 1 selected from
[3-(3,5-*bis*-trifluoromethyl-phenyl)-4-cyano-pyrrol-1-yl]-acetic acid;
[3-cyano-4-(3,5-dichloro-pheno)-pyrrol-1-yl]-acetic acid;
[3-Cyano-4-(2,2-difluoro-benzo[1,3]dioxol-4-yl)-pyrrol-1-yl-acetic acid;
(3-Cyano-4-phenyl-pyrrol-1-yl)acetic acid;
(3-Benzo[1,3]dioxol-4-yl-4-cyano-pyrrol-1-yl)-acetic acid;
[3-(2-Chloro-phenyl)-4-cyano-pyrrol-1-yl]-acetic acid;
[3-(3-Chloro-phenyl)-4-cyano-pyrrol-1-yl]-acetic acid;
[3-(4-Chloro-phenyl)-4-cyano-pyrrol-1-yl]-acetic acid;
[3-Cyano-4(4-fluoro-phenyl)-pyrrol-1-yl]-acetic acid;
[3-Cyano-4-(4-trifluoromethyl-phenyl)-pyrrol-1-yl]-acetic acid;
[3-Cyano-4-(3-trifluoromethyl-phenyl)-pyrrol-1-yl]-acetic acid;
[3-Cyano-4-(3-cyano-phenyl)-pyrrol-1-y1]-acetic acid;
[3-Cyano-4,(3,5-difluoro-phenyl)-pyrrol-1-yl]-acetic acid;
[3-Cyano-4-(2,5-dichloro-phenyl)-pyrrol-1-yl]-acetic acid;
[3-(3-Chloro-2-fluoro-phenyl)-4-cyano-pyrrol-1-yl]-acetic acid;
[3-(2-Chloro-5-trifluoromethyl-phenyl)-4-cyano-pyrrol-1-yl]-acetic acid;
[3-Cyano-4-(2-cyano-phenyl)-pyrrol-1-yl]-acetic acid;
[3-Cyano-4-(3-fluoro-phenyl)-pyrrol-1-yl]-acetic acid;
[3-Cyano-4-(2,6-dichloro-phenyl)-pyrrol-1-yl]-acetic acid;
[3-(2,5-8is-trifluoromethyl-phenyl)-4-cyano-pyrrol-1-yl]-acetic acid;
[3-(2-Chloro-3-trifluoromethyl-phenyl)-4-cyano-pyrrol-1-yl]-acetic acid;
[3-Cyano-4-(2-fluoro-3-trifluoromethyl-phenyl)-pyrrol-1-yl]-acetic acid;
[3-Cyano-4-(2-triftuoromethyl-phenyl)-pyrrol-1-yl]-acetic acid;
[3-(3-Chloro-2-fluoro-5-trifluoromethyl-phenyl)-4-cyano-pyrrol-1-yl]-acetic acid;
[3-Cyano-4-(3-fluoro-5-trifluoromethyl-phenyl)-pyrrol-yl]-acetic acid;
[3-Cyano-4-(3-trifluoromethylsulfanyl-pheny)-pyrrol-1-yl]-acetic acid;
[3-Cyano-4-(3-methanesulfonyl-phenyl)-pyrrol-1-yl]-acetic acid;
[3-(3-chloro-5-cyano-phenyl)-4-cyano-pyrrol-1-yl]-acetic acid;
[3-(3-chloro-5-trifluoromethyl-phenyl)-4-cyano-pyrrol-1-yl]-acetic acid;
(3-cyano-4-pyridin-3-yl-pyrrol-1-yl)-acetic acid;
3-cyano-4-(3,5-dimethoxy-phenyl)-pyrrol-1-yl]-acetic acid;
(3-benzofuran-2-yl-4-cyano-pyrrol-1-yl)-acetic acid;
[3-cyano-4-(3-iodo-5-trifluoromethyl-phenyl)-pyrrol-1-yl]-acetic acid;
[3-cyano-4-(3-cyano-5-trifluoromethyl-phenyl)-pyrrol-1-yl]-acetic acid;
[3-cyano-4-(3-ethanesulfonyl-5-trifluoromethyl-phenyl)-pyrrol-1-yl]-acetic acid;
{3-cyano-4-(3-(piperidine-1-sulfonyl)-5-trifluoromethyl-phenyl]-pyrrol-1-yl}-acetic acid;
{3-cyano-4-[3-(ethyl-methyl-sulfamoyl)-5-trifluoromethyl-phenyl)-pyrrol-1-yl)-acetic acid;
{3-cyano-4-[3<pyrrolidine-1-sulfonyl)-5-trifluoromethyl-phenyl]-pyrrol-1-yl}-acetic acid;
[3-cyano-4-(3-diethylsulfamoyl-5-trifluoromethyl-phenyl)-pyrrol-1-yl]-acetic acid;
(3-cyano-4-{3-[4-(2-cyano-ethyl)-piperazine-1-sulfonyl]-5-trifluoromethyl-phenyl}-pyrrol-1-yl)-acetic acid;
[3-(3-butylsulfamoyl-5-trifluoromethyl-phenyl)-4-cyano-pyrrol-1-yl]-acetic acid;
3-cyano-4-{3-[(2-cyano-ethyl)-methyl-sulfamoyl]-5-trifluoromethyl-phenyl)-pyrrol-1-yl)-acetic acid;
{3-cyano-4-[3-(morpholine-4-sulfonyl)-5-trifluoromethyl-phenyl]-pyrrol-1-yl}-acetic acid;
{3-cyano-4-[3-(2,2-dimethyl-propylsulfamoyl)-5-trifluoromethyl-phenyl]-pyrrol-1-yl}-acetic acid;
{3-[3-(butyl-methyl-sulfamoyl)-5-trifluoromethyl-phenyl]-4-cyano-pyrrol-1-yl}-acetic acid;
[3-cyano-4-(3-cyclobutylsulfamoyl-5-trifluoromethyl-phenyl)-pyrrol-1-yl]-acetic acid;
[3-cyano-4-(3-cyclohexylsulfamoyl-5-trifluoromethyl-phenyl)-pyrrol-1-yl]-acetic acid;
3-cyano-4-(3-trifluoromethanesulfonyl-phenyl)-pyrrol-1-yl]-acetic acid;
{3-[3-chloro-5-(piperidine-1-sulfonyl)-phenyl]-4-cyano-pyrrol-1-yl}-acetic acid;
{3-[3-chloro-5-(pyrrolidlne-1-sulfonyl}-phenyl]-4-cyano-pyrrol-1-yl}-acetic add; ..
3-(3-butylsulfamoyl-5-chloro-phenyl)-4-cyano-pyrrol-1-yl)-acetic acid;
{3-[3-chloro-5-(morpholine-4-sulfonyl)-phenyl]-4-cyano-pyrrol-1-yl}-acetic acid;
(3-{3-chloro-5-[4-(2-cyano-ethyl)-piperazine-1-sulfonyl]-phenyl}-4-cyano-pyrrol-1-yl)-acetic acid;
[3-(3-chloro-5-ethanesulfdnyl-pheriyo-4-cyano-pyrrol-1-yl]-acetic acid;
(3-{3-chloro-5-[(2-hydroxy-ethyl)-methyl-sulfamoyl]-phenyl}-4-cyano-pyrrol-1-yl)-acetic acid;
{3-[3-(butane-1-sulfonyl)-5-trifluoromethyl-phenyl]-4-cyano-pyrrol-1-yl}-acetic acid;
{3-cyano-4-[3-(propane-1-sulfonyl)-5-trifluoromethyl-phenyl)-pyrrol-1-yl}-acetic acid;

[3-cyano-4-(4'-fluoro-5-trifluoromethyl-biphenyl-3-yl)-pyrrol-1-yl]-acetic acid;
3-(3-chloro-5-dimethylsulfamoyl-phenyl)-4-cyano-pyrrol-1-yl]-acetic acid;
[3-cyano-4-(3-dimethylsulfamoyl-5-trifluoromethyl-phenyl)-pyrrol-1-yl]-acetic acid;
{3-cyano-4-[3-(propane-2-sulfonyl)-5-trifluoromethyl-phenyl]-pyrrol-1-yl}-acetic acid;
[3-cyano-4-(3-methanesulfonyl-5-trifluoromethylphenyl)-pyrrol-1-yl]-acetic acid; and 2-[3-(3,5-*bis*-trifluoromethyl-phenyl)-4-cyano-pyrrol-1-yl]-propionic acid.

**6.** A compound according to any one of Claims 1-5 for use as a pharmaceutical.

**7.** Pharmaceutical compositions comprising a compound according to any one of Claims 1-5.

**8.** A Compound according to any one of Claims 1-5 for use in the treatment of an inflammatory or allergic condition, particularly an inflammatory or obstructive airways disease.

**9.** A combination of a compound according to any one of claims 1-5 with an anti-inflammatory, bronchodilatory, antihistamine or anti-tussive drug substance.

**10.** A process for the preparation of compounds of formula (I) as defined in Claim 1, in free or pharmaceutically acceptable salt form, which comprises the steps of:

(i) cleaving an ester group -$COOR^7$ in a compound of formula (II)

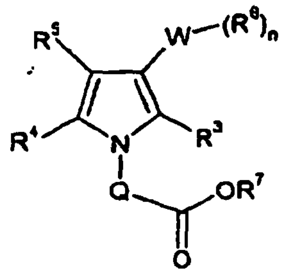

(II)

wherein
$R^7$ is $C_1$-$C_8$-alkyl; and
everything else as hereinbefore defined; and
(ii) recovering the resultant compound of formula (I), in free or pharmaceutically acceptable salt form.

## Patentansprüche

**1.** Zusammensetzung der Formel (I)

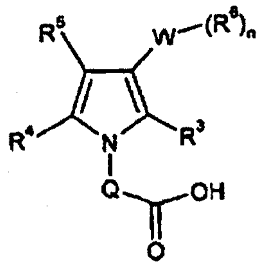

(I)

in freier oder pharmazeutisch zulässiger salziger Form,
wobei

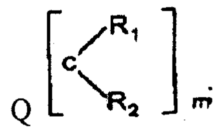

ist,

$R^1$ und $R^2$ unabhängig H, $C_1$-$C_8$-Alkyl sind oder zusammen mit dem Kohlenstoffatom, an das sie gebunden sind, eine divalente cycloaliphatische $C_3$-$C_8$-Gruppe bilden;
$R^3$ und $R^4$ unabhängig gewählt sind aus H und $C_{1-}C_8$-Alkyl;
$R^5$ Cyan ist;
$R^6$ ausgewählt ist aus H, Halogen, $C_1$-$C_8$-Alkyl, $C_1$-$C_8$-Haloalkyl, einer carbocyclischen $C_3$-$C_{15}$-Gruppe, Nitro, Cyan, $SO_2R^{6a}$, $C_1$-$C_8$-Alkylcarbonyl, $C_1$-$C_8$-Alkoxycarbonyl, $C_1$-$C_8$-Alkoxy, $C_1$-$C_8$-Haloalkoxy, -$SR^{6b}$, Carboxy, Carboxy-$C_1$-$C_8$-alkyl, Amino, Amino($C_1$-$C_8$-alkyl), $C_1$-$C_8$-Alkylamino($C_1$-$C_8$-alkyl), Di($C_1$-$C_8$-alkyl)amino ($C_1$-$C_8$-alkyl), $C_1$-$C_8$-Alkylamino, Di($C_1$-$C_8$-alkyl)amino, $SO_2NR^{6c}R^{6d}$, -C(O) $NR^{6e}R^{6f}$, $C_1$-$C_8$-Hydroxyalkyl, $NR^{6g}SO_2R^{6h}$, $NR^{6i}(CO)R^{6j}$, $SOR^{6k}$, einer aromatischen carbocyclischen $C_8$-$C_{15}$-Gruppe und einer 4- bis 10-gliedrigen heterocyklischen Gruppe, die ein oder mehrere Heteroatom(e) umfasst, welche(s) aus der Gruppe bestehend aus Sauerstoff, Stickstoff und Schwefel ausgewählt ist/sind;
$R^{6a}$, $R^{6k}$ und $R^{6b}$ unabhängig ausgewählt sind aus $C_1$-$C_8$-Alkyl, $C_1$-$C_8$-Hydroxyalkyl, $C_1$-$C_8$-Alkylamino($C_1$-$C_8$-alkyl), Di($C_1$-$C_8$-alkyl)amino($C_1$-$C_8$-alkyl), $C_{1-}C_8$-Cyanalkyl, einer carbocyclischen $C_3$-$C_{15}$-Gruppe, $C_1$-$C_8$-Haloalkyl und einer 4-bis 10-gliedrigen heterocyklischen Gruppe, die ein oder mehrere Heteroatom(e) umfasst, welche (s) aus der Gruppe bestehend aus Sauerstoff, Stickstoff und Schwefel ausgewählt ist/sind;
$R^{6c}$, $R^{6d}$, $R^{6e}$ und $R^{6f}$ unabhängig ausgewählt sind aus H, $C_1$-$C_8$-Alkyl, $C_1$-$C_8$-Hydroxyalkyl, $C_1$-$C_8$-Alkylamino ($C_1$-$C_8$-alkyl), Di($C_1$-$C_8$-alkyl)amino($C_1$-$C_8$-alkyl), $C_1$-$C_8$-Cyanalkyl, einer carbocyclischen $C_3$-$C_{15}$-Gruppe, $C_1$-$C_8$-Haloalkyl, einer 4- bis 10-gliedrigen heterocyclischen Gruppe, die ein oder mehrere Heteroatom(e) umfasst, welche(s) aus der Gruppe bestehend aus Sauerstoff, Stickstoff und Schwefel ausgewählt ist/sind, oder zusammen mit dem Stickstoffatom, an das sie gebunden sind, eine heterocyclische $C_4$-$C_{10}$-Gruppe bilden;
$R^{6g}$ und $R^{6i}$ unabhängig ausgewählt sind aus H, $C_1$-$C_8$-Alkyl, $C_1$-$C_8$-Hydroxyalkyl, $C_1$-$C_8$-Alkylamino($C_1$-$C_8$-alkyl), Di($C_1$-$C_8$-alkyl)amino($C_1$-$C_8$-alkyl), $C_1$-$C_8$-Cyanalkyl, einer carbocyclischen $C_3$-$C_{15}$-Gruppe, $C_1$-$C_8$-Haloalkyl und einer 4-bis 10-gliedrigen heterocyclischen Gruppe, die ein oder mehrere Heteroatom(e) umfasst, welche (s) aus der Gruppe bestehend aus Sauerstoff, Stickstoff und Schwefel ausgewählt ist/sind;
$^{6h}$ und $R^{6j}$ Runabhängig ausgewählt sind aus $C_1$-$C_8$-Alkyl, einer carbocyclischen $C_3$-$C_{15}$-Gruppe, einer 4- bis 10-gliedrigen heterocyclischen Gruppe, die ein oder mehrere Heteroatom(e) umfasst, welche(s) aus der Gruppe bestehend aus Sauerstoff, Stickstoff und Schwefel ausgewählt ist/sind, $C_1$-$C_8$-Hydroxyalkyl, Amino($C_1$-$C_8$-alkyl), $C_1$-$C_8$-alkylamino($C_1$-$C_8$-Alkyl), Di($C_1$-$C_8$-alkyl)amino($C_1$-$C_8$-alkyl) und $C_1$-$C_8$-Cyanalkyl;
W ausgewählt ist aus einer carbocyclischen $C_3$-$C_{15}$-Gruppe und einem 4- bis 10- gliedrigen Heterocyclus, der ein oder mehrere Heteroatom (e) umfasst, welche (s) aus der Gruppe bestehend aus Sauerstoff, Stickstoff und Schwefel ausgewählt ist/ sind;

m eine Ganzzahl von 1-3 ist; und
n eine Ganzzahl von 1-4 ist;
mit der Maßgabe, dass die Zusammensetzungen
[3-Cyan-4-(2,3-dichlor-phenyl)-pyrrol-1-yl]-Essigsäure;
N-(2-Carboxyethyl)-3-cyan-4-(2,3-dichlor-phenyl)pyrrol;
N-(3-Carboxypropyl)-3-cyan-4-(2,3-dichlor-phenyl)pyrrol; und
N-(1-Carboxyethyl)-3-cyan-4-(2,3-dichlor-phenyl)pyrrol ausgenommen sind.

**2.** Zusammensetzung der Formel (I) nach Anspruch 1, in freier oder pharmazeutisch zulässiger salziger Form, wobei die Symbole unabhängig, zusammengefasst oder in beliebiger Kombination oder Teilkombination davon folgende Bedeutungen haben:

(i) $R^1$ und $R^2$ sind unabhängig H oder $C_1$-$C_8$-Alkyl;
(ii) $R^3$ und $R^4$ sind H oder $C_1$-$C_8$-Alkyl;
(iii) $R^5$ ist Cyan;
(iv) $R^6$ ist H, Halogen, $C_1$-$C_8$-Alkyl, $C_1$-$C_8$-Haloalkyl, Cyan, $SO_2R^{6a}$ und -$SR^{6b}$;
(v) $R^{6a}$ und $R^{6b}$ sind unabhängig ausgewählt aus $C_1$-$C_8$-Alkyl, einer carbocyclischen $C_3$-$C_{15}$-Gruppe und $C_1$-$C_8$-Haloalkyl;

(vi) W ist eine carbocyclische $C_3$-$C_{10}$-Gruppe oder eine 4- bis 10-gliedrige heterocyclische Gruppe mit einem oder mehreren Sauerstoff-Ringatom(en);
(vii) m ist 1; und
(viii) n ist eine Ganzzahl von 1-4.

**3.** Zusammensetzung der Formel (I) nach Anspruch 1, in freier oder pharmazeutisch zulässiger salziger Form, wobei die Zusammensetzung die Formel (Ia) aufweist

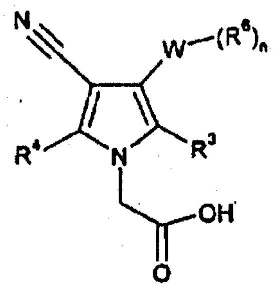

(Ia)

**4.** Zusammensetzung nach Anspruch 3, in freier oder pharmazeutisch zulässiger salziger Form, wobei
$R^3$ und $R^4$ H ist; und
$R^6$ ausgewählt ist aus H, Halogen, $C_1$-$C_8$-Haloalkyl, Cyan, $SO_2R^{6a}$ und -$SR^{6b}$;
$R^{6a}$ und $R^{6b}$ unabhängig ausgewählt sind aus $C_1$-$C_4$-Alkyl, einer carbocyclischen $C_6$-$C_{10}$-Gruppe und $C_{1-}C_4$-Haloalkyl; und
n eine Ganzzahl von 2-3 ist.

**5.** Zusammensetzung nach Anspruch 1, ausgewählt aus:

[3-(3,5-*Bis*-trifluormethyl-phenyl)-4-cyan-pyrrol-1-yl]-Essigsäure;
[3-cyan-4-(3,5-dichlor-phenyl)-pyrrol-1-yl]-Essigsäure;
[3-Cyan-4-(2,2-difluor-benzo[1,3]dioxol-4-yl)-pyrrol-1-yl-Essigsäure;
(3-Cyan-4-phenyl-pyrrol-1-yl)-Essigsäure;
(3-Benzo[1,3]dioxol-4-yl-4-cyan-pyrrol-1-yl)-Essigsäure;
[3-(2-Chlor-phenyl)-4-cyan-pyrrol-1yl]-Essigsäure;
[3-(3-Chlor-phenyl)-4-cyan-pyrrol-1-yl]-Essigsäure;
[3-(4-Chlor-phenyl)-4-cyan-pyrrol-1-yl]-Essigsäure;
[3-Cyan-4(4-fluor-phenyl)-pyrrol-1-yl]-Essigsäure;
[3-Cyan-4-(4-trifluormethyl-phenyl)-pyrrol-1-yl]-Essigsäure;
[3-Cyan-4-(3-trifluormethyl-phenyl)-pyrrol-1-yl]-Essigsäure;
[3-Cyan-4-(3-cyan-phenyl)-pyrrol-1-yl]-Essigsäure;
[3-Cyan-4-(3,5-difluor-phenyl)-pyrrol-1-yl]-Essigsäure;
[3-Cyan-4-(2,5-dichlor-phenyl)-pyrrol-1-yl]-Essigsäure;
[3-(3-Chlor-2-fluor-phenyl)-4-cyan-pyrrol-1-yl]-Essigsäure;
[3-(2-Chlor-5-trifluormethyl-phenyl)-4-cyan-pyrrol-1-yl]-Essigsäure;
[3-Cyan-4-(2-cyan-phenyl)-pyrrol-1-yl]-Essigsäure;
[3-Cyan-4-(3-fluor-phenyl)-pyrrol-1-yl]-Essigsäure;
[3-Cyan-4-(2,6-dichlor-phenyl)-pyrrol-1-yl]-Essigsäure;
[3-(2,5-Bis-trifluormethyl-phenyl)-4-cyan-pyrrol-1-yl]-Essigsäure;
[3-(2-Chlor-3-trifluormethyl-phenyl)-4-cyan-pyrrol-1-yl]-Essigsäure;
[3-Cyan-4-(2-fluor-3-trifluormethyl-phenyl)-pyrrol-1-yl]-Essigsäure;
[3-Cyan-4-(2-trifluormethyl-phenyl)-pyrrol-1-yl]-Essigsäure;
[3-(3-Chlor-2-fluor-5-trifluormethyl-phenyl)-4-cyan-pyrrol-1-yl]-Essigsäure;
[3-Cyan-4-(3-fluor-5-trifluormethyl-phenyl)-pyrrol-1-yl]-Essigsäure;
[3-Cyan-4-(3-trifluormethy lsulfanyl-phenyl)-pyrrol-1-yl]-Essigsäure;
[3-Cyan-4-(3-methansulfonyl-phenyl)-pyrrol-1-yl]-Essigsäure;
[3-(3-Chlor-5-cyan-phenyl)-4-cyan-pyrrol-1-yl]-Essigsäure;
[3-(3-Chlor-5-trifluormethyl-phenyl)-4-cyan-pyrrol-1-yl]-Essigsäure;

(3-Cyan-4-pyridin-3-yl-pyrrol-1-yl)-Essigsäure;
3-Cyan-4-(3,5-dimethoxy-phenyl)-pyrrol-1-yl]-Essigsäure;
(3-Benzofuran-2-yl-4-cyan-pyrrol-1-yl)-Essigsäure;
[3-Cyan-4-(3-iod-5-trifluormethyl-phenyl)-pyrrol-1-yl]-Essigsäure;
[3-Cyan-4-(3-cyan-5-trifluormethyl-phenyl)-pyrrol-1-yl]-Essigsäure;
[3-Cyan-4-(3-ethansulfonyl-5-trifluormethyl-phenyl)-pyrrol-1-yl]-Essigsäure;
{3-Cyan-4-[3-(piperidin-1-sulfonyl)-5-trifluormethyl-phenyl]-pyrrol-1-yl}-Essigsäure;
{3-Cyan-4-[3-(ethyl-methyl-sulfamoyl)-5-trifluormethyl-phenyl]-pyrrol-1-yl}-Essigsäure;
{3-Cyan-4-[3-(pyrrolidin-1-sulfonyl)-5-trifluormethyl-phenyl}-pyrrol-1-yl}-Essigsäure;
[3-Cyan-4-(3-diethylsulfamoyl-5-trifluormethyl-phenyl)-pyrrol-1-yl]-Essigsäure;
(3-Cyan-4-{3-[4-(2-cyan-ethyl)-piperazin-1-sulfonyl]-5-trifluormethyl-phenyl}-pyrrol-1-yl)-Essigsäure;
3-(3-Butylsulfamoyl-5-trifluormethyl-phenyl)-4-cyan-pyrrol-1-yl]-Essigsäure;
3-Cyan-4-{3-[(2-cyan-ethyl)-methyl-sulfamoyl]-5-trifluormethyl-phenyl}-pyrrol-1-yl)-Essigsäure;
{3-Cyan-4-[3-(morpholin-4-sulfonyl)-5-trifluormethyl-phenyl]-pyrrol-1-yl}-Essigsäure;
{3-Cyan-4-[3-(2,2-dimethyl-propylsulfamoyl)-5-trifluormethyl-phenyl]-pyrrol-1-yl}-Essigsäure;
{3-[3-(Butyl-methyl-sulfamoyl)-5-trifluormethyl-phenyl]-4-cyan-pyrrol-1-yl}-Essigsäure;
[3-Cyan-4-(3-cyclobutylsulfamoyl-5-trifluormethyl-phenyl)-pyrrol-1-yl]-Essigsäure;
[3-Cyan-4-(3-cyclohexylsulfamoyl-5-trifluormethyl-phenyl)-pyrrol-1-yl]-Essigsäure;
3-Cyan-4-(3-trifluormethansulfonyl-phenyl)-pyrrol-1-yl]-Essigsäure;
{3-[3-Chlor-5-(piperidin-1-sulfonyl)-phenyl]-4-cyan-pyrrol-1-yl}-Essigsäure;
{3-[3-Chlor-5-(pyrrolidin-1-sulfonyl)-phenyl]-4-cyan-pyrrol-1-yl}-Essigsäure;
3-(3-Butylsulfamoyl-5-chlor-phenyl)-4-cyan-pyrrol-1-yl]-Essigsäure;
{3-[3-Chlor-5-(morpholin-4-sulfonyl)-phenyl]-4-cyan-pyrrol-1-yl}-Essigsäure;
(3-{3-Chlor-5-[4-(2-cyan-ethyl)-piperazin-1-sulfonyl]-phenyl}-4-cyan-pyrrol-1-yl)-Essigsäure;
[3-(3-Chlor-5-ethansulfonyl-phenyl)-4-cyan-pyrrol-1-yl]-Essigsäure;
(3-{3-Chlor-5-[(2-hydroxy-ethyl)-methyl-sulfamoyl]-phenyl}-4-cyan-pyrrol-1-yl)-Essigsäure;
{3-[3-{Butan-1-sulfonyl)-5-trifluormethyl-phenyl]-4-cyan-pyrrol-1-yl}-Essigsäure;
{3-Cyan-4-[3-(propan-1-sulfonyl)-5-trifluormethyl-phenyl]-pyrrol-1-yl}-Essigsäure;
[3-Cyan-4-(4'-fluor-5-trifluormethyl-biphenyl-3-yl)-pyrrol-1-yl]-Essigsäure;
3-(3-Chlor-5-dimethylsulfamoyl-phenyl)-4-cyan-pyrrol-1-yl]-Essigsäure;
[3-Cyan-4-(3-dimethylsulfamoyl-5-trifluormethyl-phenyl)-pyrrol-1-yl]-Essigsäure;
{3-Cyan-4-[3-(propan-2-sulfonyl)-5-trifluormethyl-phenyl]-pyrrol-1-yl}-Essigsäure;
[3-Cyan-4-(3-methansulfonyl-5-trifluormethyl-phenyl)-pyrrol-1-yl]-Essigsäure; und
2-[3-(3,5-*Bis*-trifluormethyl-phenyl)-4-cyan-pyrrol-1-yl]-Propionsäure.

**6.** Zusammensetzung nach einem der Ansprüche 1-5 zur Verwendung als Arzneimittel.

**7.** Pharmazeutische Zusammensetzung, umfassend eine Zusammensetzung nach einem der Ansprüche 1-5.

**8.** Zusammensetzung nach einem der Ansprüche 1-5, zur Verwendung für die Behandlung eines entzündlichen oder allergischen Zustands, insbesondere einer entzündlichen oder obstruktiven Atemwegserkrankung.

**9.** Kombination einer Zusammensetzung nach einem der Ansprüche 1-5 mit einer entzündungshemmenden, bronchodilatorischen, Antihistamin- oder antitussiven Arzneimittelsubstanz.

**10.** Verfahren zur Zubereitung von Zusammensetzungen der Formel (I) wie in Anspruch 1 angegeben in freier oder pharmazeutisch zulässiger salziger Form, das folgende Schritte umfasst:

(i) Abspalten einer Ester-Gruppe -$COOR^7$ in einer Zusammensetzung der Formel (II)

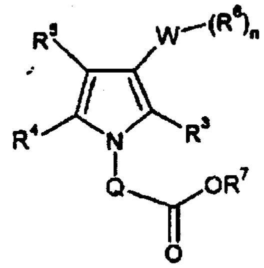

(II)

wobei
$R^7 C_1$-$C_8$-Alkyl ist; und
alle anderen wie vorstehend definiert sind; und
(ii) Gewinnen der resultierenden Zusammensetzung der Formel (I) in freier oder pharmazeutisch zulässiger salziger Form.

**Revendications**

**1.** Un composé selon la formule (I)

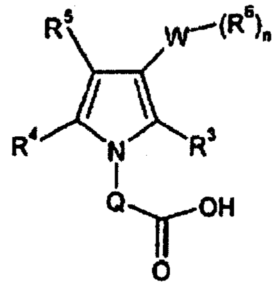

(I)

sous forme libre ou de sel pharmaceutiquement acceptable,
où
Q est

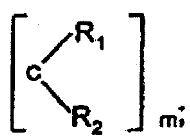

$R^1$ et $R^2$ sont, indépendamment, H, $C_1$-$C_8$-alkyle, ou conjointement avec l'atome de carbone auquel ils sont attachés forment un groupe cycloaliphatique divalent $C_3$-$C_8$,
$R^3$ et $R^4$ sont indépendamment sélectionnés parmi H et $C_1$-$C_8$-alkyle,
$R^5$ est cyano,
$R^6$ est sélectionné parmi H, halogène, $C_1$-$C_8$-alkyle, $C_1$-$C_8$-haloalkyle, un groupe carbocyclique $C_3$-$C_{15}$, nitro, cyano, $SO_2R^{6a}$, $C_1$-$C_8$-alkylcarbonyle, $C_1$-$C_8$-alkoxycarbonyle, $C_1$-$C_8$-alkoxy, $C_1$-$C_8$-haloalkoxy, $-SR^{6b}$, carboxy, carboxy-$C_1$-$C_8$-alkyle, amino, amino($C_1$-$C_8$-alkyle), $C_1$-$C_8$-alkylamino($C_1$-$C_8$-alkyle), di($C_1$-$C_8$-alkyle)amino($C_1$-$C_8$-alkyle), $C_1$-$C_8$-alkylamino, di($C_1$-$C_8$-alkyle)amino, $SO_2NR^{6c}R^{6d}$, $-C(O)NR^{6e}R^{6f}$, $C_1$-$C_8$-hydroxyalkyle, $NR^{6g}SO_2R^{6h}$, $NR^{6i}(CO)R^{6j}$, $SOR^{6k}$, un groupe carbocyclique aromatique $C_6$-$C_{15}$ et un groupe hétérocyclique de 4 à 10 chaînons possédant un ou plusieurs hétéroatomes sélectionnés dans le groupe se composant d'oxygène, d'azote et de soufre,
$R^{6a}$, $R^{6k}$ et $R^{6b}$ sont indépendamment sélectionnés parmi $C_1$-$C_8$-alkyle, $C_1$-$C_8$-hydroxyalkyle, $C_1$-$C_8$-alkylamino ($C_1$-$C_8$-alkyle), di($C_1$-$C_8$-alkyle)amino($C_1$-$C_8$-alkyle), $C_1$-$C_8$-cyanoalkyle, un groupe carbocyclique $C_3$-$C_{15}$, $C_1$-$C_8$-haloalkyle et un groupe hétérocyclique de 4 à 10 chaînons possédant un ou plusieurs hétéroatomes

sélectionnés dans le groupe se composant d'oxygène, d'azote et de soufre,
$R^{6c}$, $R^{6d}$, $R^{6e}$ et $R^{6f}$ sont indépendamment H, $C_1$-$C_8$-alkyle, $C_1$-$C_8$-hydroxyalkyle, $C_1$-$C_8$-alkylamino($C_1$-$C_8$-alkyle), di($C_1$-$C_8$-alkyle)amino($C_1$-$C_8$-alkyle), $C_1$-$C_8$-cyanoalkyle, un groupe carbocyclique $C_3$-$C_{15}$, $C_1$-$C_8$-haloalkyle, un groupe hétérocyclique de 4 à 10 chaînons possédant un ou plusieurs hétéroatomes sélectionnés dans le groupe se composant d'oxygène, d'azote et de soufre, ou conjointement avec l'atome d'azote auquel ils sont attachés forment un groupe hétérocyclique $C_4$-$C_{10}$,
$R^{6g}$ et $R^{6l}$ sont indépendamment sélectionnés parmi H, $C_1$-$C_8$-alkyle, $C_1$-$C_8$-hydroxyalkyle, $C_1$-$C_8$-alkylamino ($C_1$-$C_8$-alkyle), di($C_1$-$C_8$-alkyle)amino($C_1$-$C_8$-alkyle), $C_1$-$C_8$-cyanoalkyle, un groupe carbocyclique $C_3$-$C_{15}$, $C_1$-$C_8$-haloalkyle et un groupe hétérocyclique de 4 à 10 chaînons possédant un ou plusieurs hétéroatomes sélectionnés dans le groupe se composant d'oxygène, d'azote et de soufre,
$R^{6h}$ et $R^{6j}$ sont indépendamment sélectionnés parmi $C_1$-$C_8$-alkyle, un groupe carbocyclique $C_3$-$C_{15,}$ un groupe hétérocyclique de 4 à 10 chaînons possédant un ou plusieurs hétéroatomes sélectionnés dans le groupe se composant d'oxygène, d'azote et de soufre, $C_1$-$C_8$-hydroxyalkyle, amino($C_1$-$C_8$ alkyle), $C_1$-$C_8$-alkylamino ($C_1$-$C_8$-alkyle), di($C_1$-$C_8$-alkyle)amino($C_1$-$C_8$-alkyle), et $C_1$-$C_8$-cyanoalkyle,

W est sélectionné parmi un groupe carbocyclique $C_3$-$C_{15}$ et un hétérocycle de 4 à 10 chaînons possédant un ou plusieurs hétéroatomes sélectionnés dans le groupe se composant d'oxygène, d'azote et de soufre,
m est un entier sélectionné de 1 à 3, et
n est un entier sélectionné de 1 à 4,
à condition que les composés
[3-cyano-4-(2,3-dichloro-phényle)-pyrrole-1-yl]-acide acétique,
N-(2-carboxyéthyle)-3-cyano-4-(2,3-dichloro-phényle)-pyrrole,
N-(3-carboxypropyle)-3-cyano-4-(2,3-dichloro-phényle)-pyrrole et
N-(1-carboxyéthyle)-3-cyano-4-(2,3-dichloro-phényle)-pyrrole soient exclus.

**2.** Un composé selon la formule (I) selon la revendication 1, sous forme libre ou de sel pharmaceutiquement acceptable,
où les symboles ont les significations suivantes, indépendamment, collectivement ou dans toute combinaison ou sous-combinaison :

(i) $R^1$ et $R^2$ sont, indépendamment, H ou $C_1$-$C_8$-alkyle,
(ii) $R^3$ et $R^4$ sont H ou $C_1$-$C_8$-alkyle,
(iii) $R^5$ est cyano,
(iv) $R^6$ est H, halogène, $C_1$-$C_8$-alkyle, $C_1$-$C_8$-haloalkyle, cyano, $SO_2R^{6a}$ et - $SR^{6b}$,
(v) $R^{6a}$ et $R^{6b}$ sont indépendamment sélectionnés parmi $C_1$-$C_8$-alkyle, un groupe carbocyclique $C_3$-$C_{15}$ et $C_1$-$C_8$-haloalkyle,
(vi) W est un groupe carbocyclique $C_3$-$C_{10}$ ou un groupe hétérocyclique de 4 à 10 chaînons possédant un ou plusieurs atomes d'oxygène d'anneau,
(vii) m est 1, et
(viii) n est un entier sélectionné de 1 à 4.

**3.** Un composé selon la revendication 1, sous forme libre ou de sel pharmaceutiquement acceptable, où le composé est de la formule (Ia)

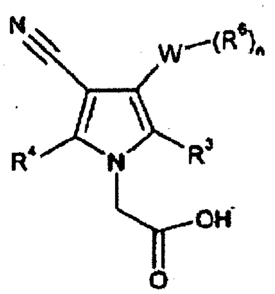

(Ia)

**4.** Un composé selon la revendication 3, sous forme libre ou de sel pharmaceutiquement acceptable, où
$R^3$ et $R^4$ sont H, et

$R^6$ est sélectionné parmi H, halogène, $C_1$-$C_8$-haloalkyle, cyano, $SO_2R^{6a}$ et - $SR^{6b}$,
$R^{6a}$ et $R^{6b}$ sont indépendamment sélectionnés parmi $C_1$-$C_4$-alkyle, un groupe carbocyclique $C_6$-$C_{10}$ et $C_1$-$C_4$-haloalkyle, et
n est un entier sélectionné parmi 2 ou 3.

**5.** Un composé selon la revendication 1 sélectionné parmi

[3-(3,5-*bis*-trifluorométhyle-phényle)-4-cyano-pyrrole-1-yl]-acide acétique,
[3-cyano-4-(3,5-dichloro-phényle)-pyrrole-1-yl]-acide acétique,
[3-Cyano-4-(2,2-difluoro-benzo[1,3]dioxole-4-yl)-pyrrole-1-yl-acide acétique,
[3-Cyano-4-phényle-pyrrole-1-yl)acide acétique,
(3-Benzo[1,3]dioxole-4-yl-4-cyano-pyrrole-1-yl)-acidé acétique,
[3-(2-Chloro-phényle)-4-cyano-pyrrole-1-yl]-acide acétique,
[3-(3-Chloro-phényle)-4-cyano-pyrrole-1-yl]-acide acétique,
[3-(4-Chloro-phényle)-4-cyano-pyrrole-1-yl]-acide acétique,
[3-Cyano-4(4-fluoro-phényle)--pyrrole-1-yl]-acide acétique,
[3-Cyano-4-(4-trifluorométhyle-phényle)-pyrrole-1-yl]-acide acétique,
[3-Cyano-4-(3-trifluorométhyle-phényle)-pyrrole-1-yl]-acide acétique,
[3-Cyano-4-(3-cyano-phényle)-pyrrole-1-yl]-acide acétique,
[3-Cyano-4-(3,5-difluoro-phényle)-pyrrole-1-yl]-acide acétique,
[3-Cyano-4-(2,5-dichloro-phényle)-pyrrole-1-yl]-acide acétique,
[3-(3-Chloro-2-fluoro-phényle)-4-cyano-pyrrole-1-yl]-acide acétique,
[3-(2-Chloro-5-trifluorométhyle-phényle)-4-cyano-pyrrole-1-yl]-acide acétique,
[3-Cyano-4-(2-cyano-phényle)-pyrrole-1-yl]-acidé acétique,
[3-Cyano-4-(3-fluoro-phényle)-pyrrole-1-yl]-acide acétique,
[3-Cyano-4-(2,6-dichloro-phényle)-pyrrole-1-yl]-acide acétique,
[3-(2,5-Bis-trifluorométhyle-phényle)-4-cyano-pyrrole-1-yl]-acide acétique,
[3-(2-Chloro-3-trifluorométhyle-phényle)-4-cyano-pyrrole-l-yl]-acide acétique,
[3-Cyano-4-(2-fluoro-3-trifluorométhyle-phényle)-pyrrole-1-yl]-acide acétique,
[3-Cyano-4-(2-trifluorométhyle-phényle)-pyrrole-1-yl]-acide acétique,
[3-(3-Chloro-2-fluoro-5-trifluorométhyle-phényle)-4-cyano-pyrrole-1-yl]-acide acétique,
[3-Cyano-4-(3-fluoro-5-trifluorométhyle-phényle)-pyrrole-1-yl]-acide acétique,
[3-Cyano-4-(3-trifluorométhylesulfanyle-phényle)-pyrrole-1-yl]-acide acétique,
[3-Cyano-4-(3-méthanesulfanyle-phényle)-pyrrole-1-yl]-acide acétique,
[3-(3-chloro-5-cyano-phényle)-4-cyano-pyrrole-1-yl]-acide acétique,
[3-(3-chloro-5-trifluorométhyle-phényle)-4-cyano-pyrrole-1-yl]-acide acétique,
(3-cyano-4-pyridine-3-yl-pyrrole-1-yl)-acide acétique,
3-cyano-4-(3,5-diméthoxy-phényle)-pyrrole-1-yl]-acide acétique,
(3-benzofurane-2-yl-4-cyano-pyrrole-1-yl)-acide acétique,
[3-cyano-4-(3-iodo-5-trifluorométhyle-phényle)-pyrrole- -yl]-acide acétique,
[3-cyano-4-(3-cyano-5-trifluorométhyle-phényle)-pyrrole-1-yl]-acide acétique,
[3-cyano-4-(3-éthanesulfonyle-5-trifluorométhyle-phényle)-pyrrole-1-yl]-acide acétique,
{3-cyano-4-[3-(piperidine-1-sulfonyle)-5-trifluorométhyle-phényle]-pyrrole-1-yl}-acide acétique,
{3-cyano-4-[3-(éthyle-méthyle-sulfamoyle)-5-trifluorométhyle-phényle]-pyrrole-1-yl}-acide acétique,
{3-cyano-4-[3-(pyrrolidine-1-sulfonyle)-5-trifluorométhyle-phényle]-pyrrole-1-yl}-acide acétique,
[3-cyano-4-(3-diéthylesulfamoyle-5-trifluorométhyle-phényle)-pyrrole-1-yl]-acide acétique,
(3-cyano-4-{3-[4-(2-cyano-éthyle)-pipérazine-1-sulfonyle]-5-trifluorométhyle-phénate}-pyrrole-1-yl)-acide acétique,
[3-(3-butylesulfamoyle-5-trifluorométhyle-phényle)-4-cyano-pyrrole-1-yl]-acide acétique,
3-cyano-4-{3-[(2-cyano-éthyle)-méthyle-sulfamoyle]-5-trifluorométhyle-phényle}-pyrrole-1-yl)-acide acétique,
{3-cyano-4-[3-(morpholine-4-sulfonyle)-5-trifluorométhyle-phényle]-pyrrole-1-yl}-acide acétique,
{3-cyano-4-[3-(2,2-diméthyle-propylesulfamoyle)-5-trifluorométhyle-phényle]-pyrrole-1-yl}-acide acétique,
{3-[3-(butyle-méthyle-sulfamoyle)-5-trifluorométhyle-phényle]-4-cyano-pyrrole-1-yl}-acide acétique,
[3-cyano-4-(3-cyclobutylesulfarnoyle-5-trifluorométhyle-phényle)-pyrrole-1-yl]-acide acétique,
[3-cyano-4-(3-cyclohexylesulfamoyle-5-trifluorométhyle-phényle)-pyrrole-1-yl]-acide acétique,
3-cyano-4-(3-trifluorométhanesulfonyle-phényle)-pyrrole-1-yl]-acide acétique,
{3-[3-chloro-5-(piperidine-1-sulfonyle)-phényle]-4-cyano-pyrrole-1-yl}-acide acétique,
{3-[3-chloro-5-(pyrrolidine-1-sulfonyle)-phényle]-4-cyano-pyrrole-1-yl}-acide acétique,

3-(3-butylesulfamoyle-5-chloro-phényle)-4-cyano-pyrrole-1-yl]-acide acétique,
(3-{3-chloro-5-(morpholine-4-sulfonyle)-phényle]-4-cyano-pyrrole-1-yl}-acide acétique,
(3-{3-chloro-5-[4-(2-cyano-éthyle)-pipérazine-1-sulfonyle]-phényle}-4-cyano-pyrrole-1-yl)-acide acétique,
[3-(3-chloro-5-éthanesulfonyle-phényle)-4-cyano-pyrrole-1-yl]-acide acétique,
(3-{3-chloro-5-[(2-hydroxy-éthyle)-méthyle-sulfamoyle]-phényle}-4-cyano-pyrrole-1-yl)-acide acétique,
{3-[3-(butane-1-sulfonyle)-5-trifluorométhyle-phényle]-4-cyano-pyrrole-1-yl}-acide acétique,
{3-cyano-4-[3-(propane-1-sulfonyle)-5-trifluorométhyle-phényle]-pyrrole-1-yl} -acide acétique,
[3-cyano-4-(4'-fluoro-5-trifluorométhyle-biphényle-3-yl)-pyrrole-1-yl]-acide acétique,
3-(3-chloro-5-diméthylesulfamoyle-phényle)-4-cyano-pyrrole-1-yl]-acide acétique,
[3-cyano-4-(3-diméthylesulfamoyle-5-trifluorométhyle-phényle)-pyrrole-1-yl]-acide acétique,
{3-cyano-4-[3-(propane-2-sulfonyle)-5-trifluorométhyle-phényle]-pyrrole-1-yl} -acide acétique,
[3-cyano-4-(3-méthanesulfonyle-5-trifluorométhyle-phényle)-pyrrole-1-yl]-acide acétique, et
2-[3-(3,5-*bis*-trifluorométhyle-phényle)-4-cyano-pyrrole-1-yl]-acide propionique.

**6.** Un composé selon l'une quelconque des revendications 1 à 5 destiné à un usage en tant que produit pharmaceutique.

**7.** Des compositions pharmaceutiques comprenant un composé selon l'une quelconque des revendications 1 à 5.

**8.** Un composé selon l'une quelconque des revendications 1 à 5 pour une utilisation dans le traitement d'un état inflammatoire ou allergique, en particulier un syndrome obstructif ou inflammatoire des voies aériennes.

**9.** Une combinaison d'un composé selon l'une quelconque des revendications 1 à 5 avec une substance médicamenteuse anti-inflammatoire, bronchodilatatrice, antihistaminique ou antitussive.

**10.** Un processus destiné à la préparation de composés de la formule (I) tels que définis à la revendication 1, sous forme libre ou de sel pharmaceutiquement acceptable, qui comprend les opérations suivantes :

(i) le clivage d'un groupe ester -COOR$^7$ dans un composé de la formule (II)

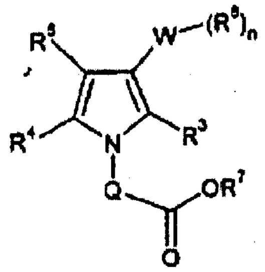

(II)

où
R$^7$ est $C_1$-$C_8$-alkyle, et
tout le reste est tel que défini ci-dessus, et
(ii) la récupération du composé résultant de la formule (I), sous forme libre ou de sel pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03101981 A
- WO 2005116001 A
- EP 612723 A
- WO 2005037196 A
- WO 2004014382 A
- WO 20041014382 A
- WO 0288167 A
- WO 0212266 A
- WO 02100879 A
- WO 0200679 A
- WO 03035668 A
- WO 03048181 A
- WO 03062259 A
- WO 03064445 A
- WO 03072592 A
- WO 04039827 A
- WO 04066920 A
- WO 0000531 A
- WO 0210143 A
- DE 10261874
- WO 03082280 A
- WO 03082787 A
- WO 03104195 A
- WO 03101932 A
- WO 04019935 A
- WO 04018429 A
- WO 04063163 A
- WO 04005229 A
- WO 03086294 A
- WO 0426248 A
- WO 04071389 A
- US 5451700 A
- WO 03104204 A
- WO 03104205 A
- WO 04000814 A
- WO 04000839 A
- WO 04005258 A
- WO 9818796 A
- WO 0339544 A
- EP 1052264 A
- EP 1241176 A
- EP 409595 A2
- WO 9417090 A
- WO 9602543 A
- WO 9602553 A
- WO 9828319 A
- WO 9924449 A
- WO 9924450 A
- WO 9924451 A
- WO 9936877 A
- WO 9941267 A
- WO 9967263 A
- WO 9967264 A
- WO 9967265 A
- WO 9967266 A
- WO 0023457 A
- WO 0077018 A
- WO 0078774 A
- WO 0123399 A
- WO 0127130 A
- WO 0127131 A
- WO 0160835 A
- WO 0194368 A
- WO 0200676 A
- WO 0222630 A
- WO 0296462 A
- WO 03086408 A
- WO 0242298 A
- WO 0075114 A
- WO 0416601 A
- JP 05025045 B
- WO 9318007 A
- WO 9964035 A
- US 20020055651 A
- WO 0142193 A
- WO 0183462 A
- WO 0266422 A
- WO 0270490 A
- WO 0276933 A
- WO 03024439 A
- WO 03072539 A
- WO 03042160 A
- WO 03091204 A
- WO 03042164 A
- WO 03099764 A
- WO 04016578 A
- WO 04022547 A
- WO 04032921 A
- WO 04037773 A
- WO 04037807 A
- WO 04039762 A
- WO 04039766 A
- WO 04045618 A
- WO 04046083 A
- WO 04033412 A
- WO 04037768 A
- EP 1440966 A
- WO 04096800 A

- WO 0104118 A
- WO 0251841 A
- WO 0253564 A
- WO 0300840 A
- WO 0387094 A
- WO 0405285 A
- WO 0200652 A
- WO 0353966 A
- EP 0424021 A
- US 5171744 A
- US 3714357 A
- WO 0333495 A
- WO 02026723 A
- WO 03077907 A
- WO 03007939 A
- WO 02102775 A
- US 6166037 A
- WO 0066558 A
- WO 0066559 A


**Non-patent literature cited in the description**


- *PEST MANAGEMENT SCIENCE,* 2004, vol. 60, 1063
- March's Organic Chemistry. Wiley and Chichester, 2001
- Comprehensive Organic Transformations. VCH, 1989
- Comprehensive Organic Functional Group Transformations. Pergamon, 1995
- Comprehensive Organic Synthesis. Pergamon, 1991
- **Pavri ; Trudell.** *J Orgr Chem,* 1997, vol. 62 (8), 2649-2651
- *Bioorg Med Chem,* 2004, vol. 12 (9), 2021-2034
- *J Med Chem,* 1989, vol. 32, 2436-2442
- **E. E. Gilbert.** *Synthesis,* 1969, vol. 1-10, 6
- **Szarka et al.** *J Immunol Methods,* 1997, vol. 202, 49-57
- **Renzi et al.** *Am Rev Respir Dis,* 1993, vol. 148, 932-939
- **Tsuyuki et al.** *J Clin Invest,* 1995, vol. 96, 2924-2931
- **Cemadas et al.** *Am J Respir Cell Mol Biol,* 1999, vol. 20, 1-8
- **Williams ; Galli.** *J Exp Med,* 2000, vol. 192, 455-462